(19) Europäisches Patentamt
European Patent Office
Office européen des brevets

(11) **EP 3 693 474 A1**

(12) **EUROPEAN PATENT APPLICATION**

(43) Date of publication:
**12.08.2020 Bulletin 2020/33**

(51) Int Cl.:
***C12Q 1/6883*** (2018.01)    ***G01N 33/569*** (2006.01)

(21) Application number: **19382093.3**

(22) Date of filing: **11.02.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Bioithas S.L.**
**03430 Onil (Alicante) (ES)**

• **Bionou Research, S.L.**
**03540 Sant Joan de Alicante (ES)**

(72) Inventor: **NAVARRO LÓPEZ, Vicente Manuel**
**03430 Onil (Alicante) (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **METHODS FOR  PREDICTING THE RISK OF PROGRESSION AND PHARMACOLOGICAL RESPONSE OF A HUMAN SUBJECT SUFFERING FROM MULTIPLE SCLEROSIS**

(57)    The present invention relates to a biomarker or to a combination of biomarkers in stool samples which on one hand help in the diagnosis of multiple sclerosis, and on the other hand predict the progression of multiple sclerosis, as well as the response to treatment of a subject suffering from this disease.

## Description

## Technical Field of the Invention

The present invention relates to a biomarker or to a combination of biomarkers in intestinal or stool samples which on one hand help in the diagnosis of multiple sclerosis, and on the other hand predict the progression of multiple sclerosis, as well as the response to treatment of a subject suffering from this disease.

## State of the Art

Multiple sclerosis (MS) is a chronic neuroinflammatory disease that affects more than 2 million people worldwide, mainly women. Although it can occur at any age, it is usually diagnosed between 25 and 30 years of age, representing the second cause of disability in young adults. The prevalence of the disease is highest in the northern and southern hemispheres and lowest at the equator, with Europe, northern United States, southern Australia, and New Zealand being the geographical areas with the highest prevalence. In Spain, it is estimated that there are 46,000 people diagnosed with MS and 5,000 in the Autonomous Community of Valencia. Etiology of the disease is unknown though it is believed that the combination of a genetic predisposition and exposure to an environmental factor may trigger the activation of an immune response against the nervous system (NS) mediated by myelin-specific autoreactive T-CD4+ lymphocytes. The external agent may be of an infectious type (e.g., Epstein-Barr virus), although other factors such as smoking, low vitamin D levels, and obesity in adolescence have been studied. Relapsing-remitting multiple sclerosis (RRMS) is the most common clinical form of multiple sclerosis. In relapsing-remitting multiple sclerosis, symptoms appear in acute episodes called "flare-ups" alternating with periods of stability. These flare-ups can leave behind significant physical consequences. The symptoms comprise sensory disorders (tingling), optic neuritis, diplopia, spasticity, coordination disorders, and other dysfunctions. Pharmacological treatments have significantly evolved, yet none of them is capable of curing the disease. Corticoids are the most frequently used in managing flare-ups, while immunomodulatory medicaments are used to slow down their progression.

As a result of the development of advanced identification techniques in molecular microbiology, virtually the entire intestinal microbial community, its functionality, and its metabolic capacities can be studied today. As a consequence, various lines of research aimed at understanding the role of human microbiome in health have emerged in the last decade. Specifically, in the field of demyelinating pathologies, there are various publications linking gut microbiota to the onset of experimental autoimmune encephalomyelitis (EAE) in mice. With regard to its relationship with MS, scientific evidence is still scarce, although some studies with patients diagnosed with RRMS and their comparison with healthy controls are already available. In general, all these studies reported intestinal dysbiosis in subjects with RRMS. Cantarel et al. (USA, 2014) analyzed the intestinal microbial diversity of 7 patients with RRMS and 8 healthy controls to measure the effect of vitamin D supplementation, with an increase in the abundance of Faecalibacterium (which is attributed anti-inflammatory effects due to its role in the production of butyrate) being observed after the intake of this vitamin. In a study conducted by Jangi et al. (Jangi S, Gandhi R, Cox LM, Li N, von Glehn F, Yan R, Patel B, Mazzola MA, Liu S, Glanz BL, et al. Alterations of the human gut microbiome in multiple sclerosis. Nat Commun. 2016 Jun 28; 7: 12015. doi: 10.1038/ncomms12015), the existence of differences at the genus level in patients with RRMS was verified when comparing samples from 60 patients with those from 43 healthy patients, in addition to establishing relationships with their serological profiles. There was an increase in Methanobrevibacter and Akkermansia, as well as a decrease in Butyricimonas and Prevotella in subjects with RRMS. Chen et al. (USA, 2016) studied samples from 31 patients with RRMS and 36 healthy individuals, with differences in the abundance of 35 taxa being observed in the four main phyla, highlighting the decrease in Prevotella and the Bacteroidetes phylum in patients with RRMS. There are other studies such as the one conducted by Tremlett et al. (Tremlett H, Fadrosh DW, Faruqi AA, et al., Gut microbiota in early pediatric multiple sclerosis: a case-control study. Eur J Neurol, 2016 Aug; 23 (8): 1308-1321, doi: 10.1111/Jan.13026. Epub 2016 May 13. PubMed PMID: 27176462; PubMed Central PMCID: PMC4955679) in pediatric patients with RRMS, which indicates a relationship between the risk of the onset of flare-ups and the absence of Fusobacteria. The methodology used in all of them was the sequencing of the 16S rRNA gene, with the amplification of the V4 region or the V3-5 region.

## Brief Description of the Drawings:

**Figure 1. Dependence of the Shannon index for both the group of healthy individuals (control) and patients with RRMS.** Species biodiversity in the control group is greater than in the treatment group.

**Figures 2a and 2b. Comparison of the microbiota composition data by family and genus between the group**

**of healthy individuals and patients with RRMS.** The bars represent the mean values of the genus with statistically significant differences (p <0.05) between the two population groups.

**Figure 3. Comparative analysis of the main microbiota components in the healthy population and in patients with RRMS.** Group samples and comparisons based on the variability of the bacterial composition at the genus taxonomic level for each patient with RRMS and the healthy population.

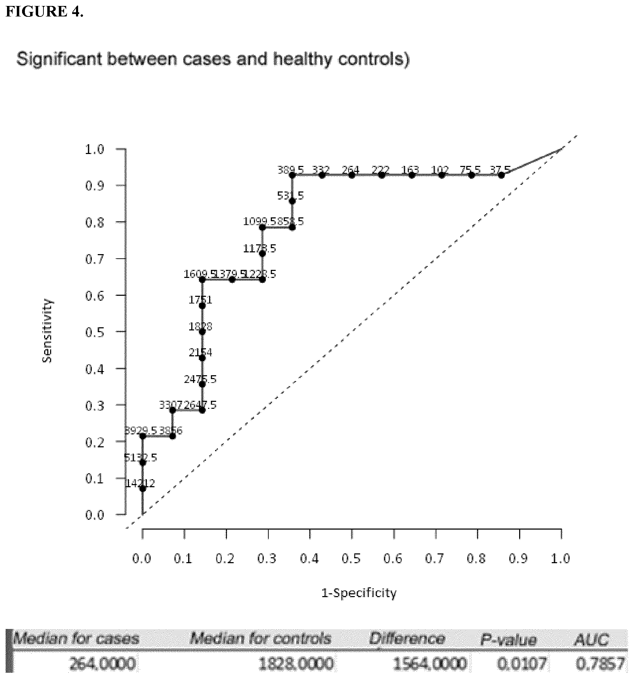

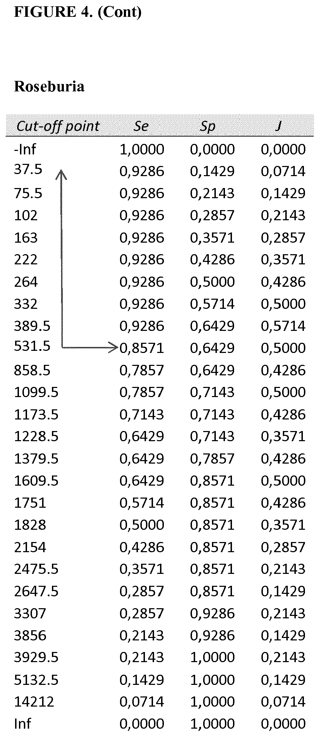

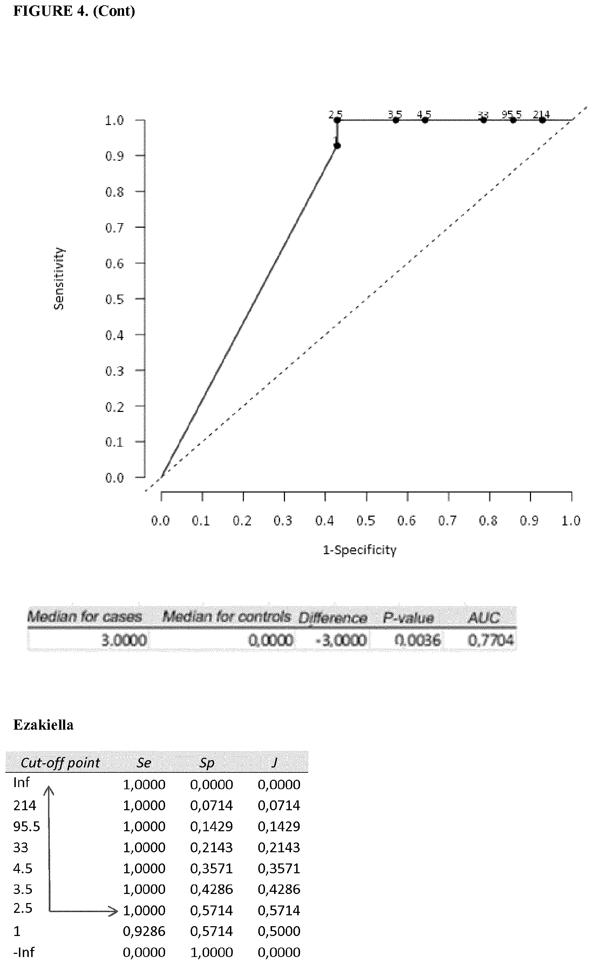

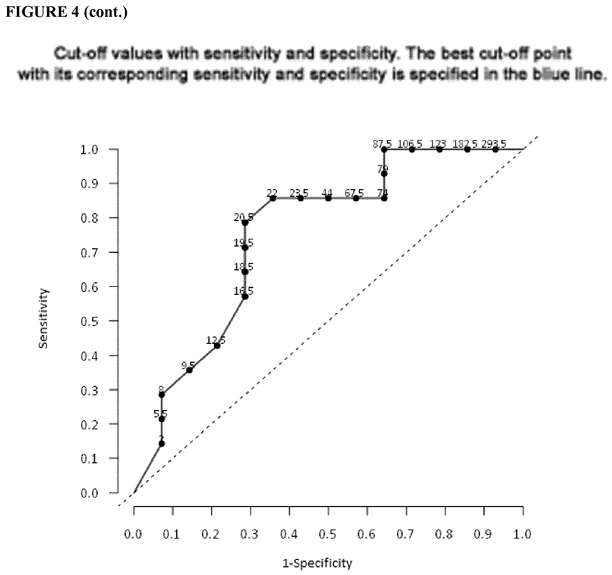

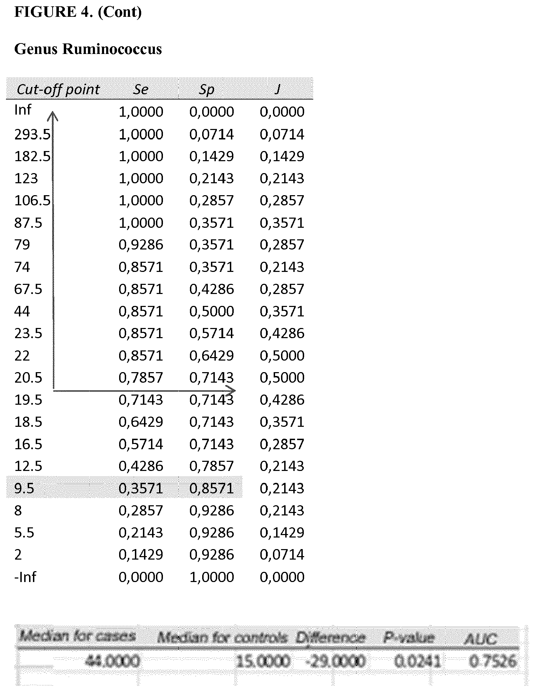

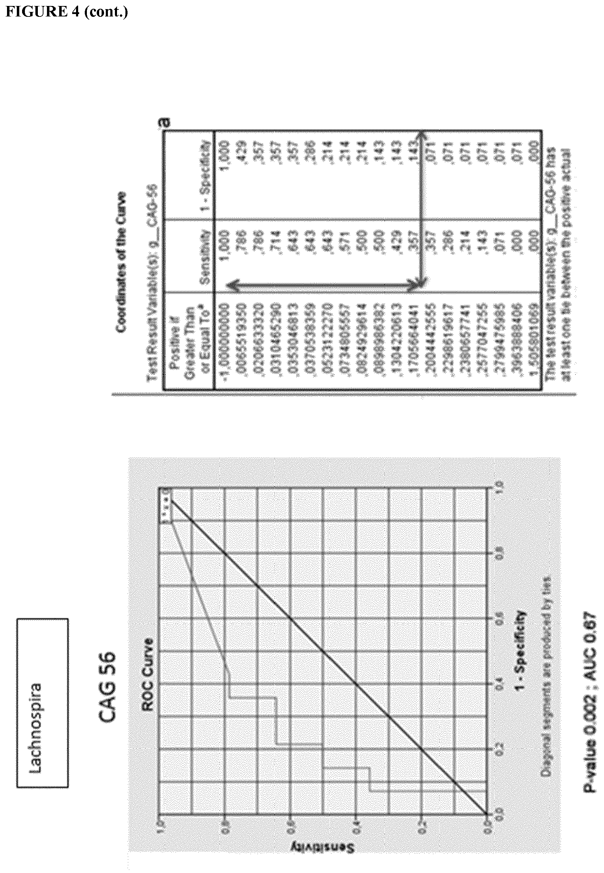

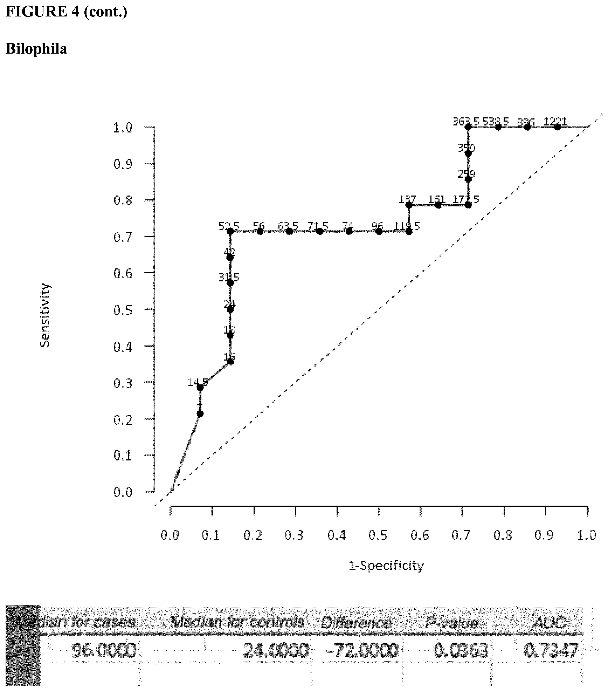

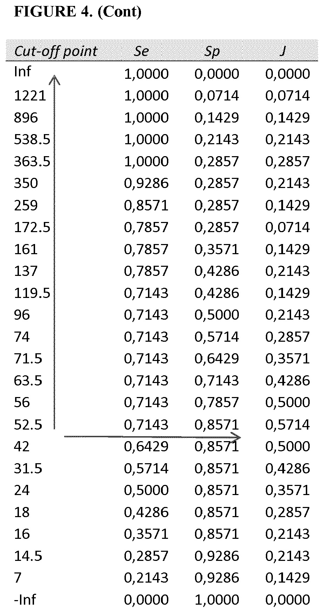

**Figure 4. Receiver operating characteristic curves (ROC curves) for calculating area under the curve and cut-off points for sensitivity and specificity for differentiating between case (patient with multiple sclerosis) and control (healthy subject) in those microorganisms in which a _significant_ association is observed at the genus level** The values of significance, cut-off values, and the area under the curve are shown in each figure.

**Figure 5. Diet record of patients with RRMS and healthy controls.** Percentage of the main components of the diet, represented as the intake frequency, recorded in the group of patients with active RRMS included in the study.

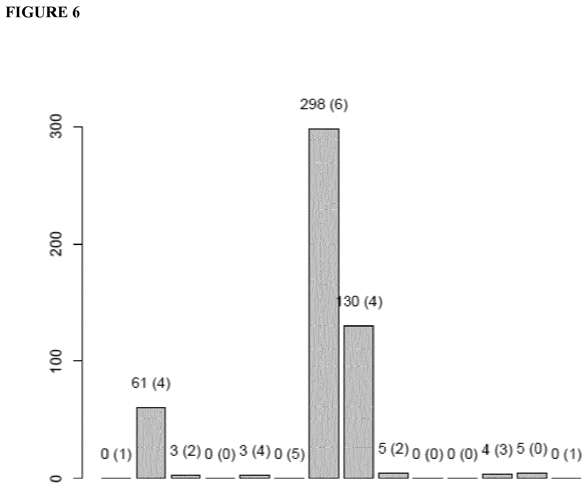

**Figure 6. Poisson analysis for evaluating disease flare-ups and their relationship with gut microbiota.** Risk of there being more flare-ups as the presence of _Ezaquiella_ as microbial composition in the stool of patients increases.

**Detailed Description of the Invention:**

It has been found in the present invention that some of the components of gut microbiota composition are capable of exhibiting differences between healthy subjects without multiple sclerosis and subjects with multiple sclerosis, and furthermore, some of these components are specifically correlated with the progression of multiple sclerosis or its response to treatment.

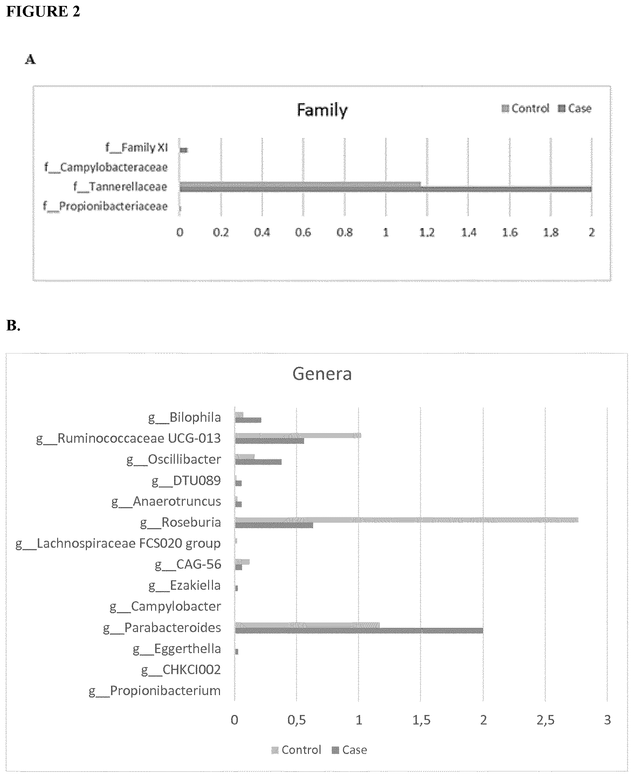

In this sense, as illustrated in the examples of the present invention the authors of the present invention have verified the existence of differences at the genus level of certain microorganisms in patients with RRMS when comparing samples from said patients with those from healthy individuals (see Figure 2b). Moreover, it was subsequently verified that not all of said genera of microorganisms for which differences between patients and healthy individuals have been determined necessarily provided information which would allow the prognosis of the progression of the course of the disease (multiple sclerosis), i.e., information which would allow predicting the number of relapses in a subject suffering from said disease or determining whether or not said subject was responding to treatment.

Nevertheless, a part of these components identified in intestinal or stool samples from those patients, that were furthermore capable of exhibiting differences between healthy subjects without multiple sclerosis and subjects with multiple sclerosis, were specifically correlated with the progression of multiple sclerosis or its response to treatment. In summary, as shown in detail in the examples and drawings of the present invention, in order to arrive at the results of the present invention the authors investigated the gut microbiota composition in a cohort of 15 individuals with RRMS using 16s rRNA sequencing. Data from the partners of the patients was used as control group because they shared the same diet and biases that diet have on the gut microbial composition were thereby prevented as demonstrated when analyzing the components of the diet and as reflected in the drawings section herein. It must be noted that the microbiota of patients with RRMS differs from the healthy population. In fact, the present invention shows the existence of a significant change in the biodiversity and composition of microorganisms in stool of these patients with respect to the control group consisting of healthy subjects.

In a second phase of the study and once it has been proven that gut microbiota is partially different between a patient with multiple sclerosis and a healthy subject, follow-up was conducted on the group of patients with multiple sclerosis over time. Furthermore, after a follow-up period for the treated patients with multiple sclerosis of at least 3 years, a correlation was observed between certain aspects of the microbiota and the onset of new flare-ups and/ or new lesions in the central nervous system (CNS). In this sense, it was observed that patients undergoing treatment with a worse clinical progression and worse response to treatment exhibited microbiota that is different from the microbiota of patients with MS who did respond to treatment. Curiously, those microorganisms that conferred a worse prognosis were furthermore present among those microorganisms found at different concentrations between healthy and sick populations. In other words, these germs seem to be involved in the development or onset of multiple sclerosis, and furthermore, part of them would be involved in a worse progression of the disease despite proper treatment.

In that sense and as illustrated in the examples of the present invention, significant differences were found when comparing cases of multiple sclerosis and healthy controls at the levels of bacteria _belonging to the genera Ezakiella, Lachnospira, **Bilophila,** Ruminococcus,_ and _Roseburia_. Moreover, when analyzing only cases of multiple sclerosis and the correlation of onset of new flare-ups with the different microorganisms, it is found that when comparing the levels of bacteria _of the genus Ezakiella,_ there was a positive correlation between the poor progression of the disease and the levels or the concentrations of those bacteria belonging to this genus. Specifically, it was found that for every 100 units of increase in the sequences of *Ezakiella,* the increase in the risk of flare-ups went up to 1.56 in comparison with patients with zero flare-ups.

Therefore, on one hand these microorganisms appear in different proportions in patients at the time of the diagnosis of multiple sclerosis, and on the other hand a worse prognosis of the disease with a poor response to treatment is observed when these microorganisms are altered with respect to the general population (specifically when there are higher levels of *Ezakiella*). All this is indicative of the role of the microbiota composition as a factor for predicting the progression of the disease and as an adjunctive test for the diagnosis of multiple sclerosis.

Once this has been done and using the selected germs, a final study of the ROC curve and the area under the curve was performed to define a cut-off value or point for each specific germ, including sensitivity and specificity, which would be the value for predicting whether or not the subject has multiple sclerosis. This data is shown in the drawings and examples.

It can be concluded based on this information that those patients diagnosed with MS have microbiota that is different from the general population despite sharing the same diet. Specifically, the genera *Ezakiella, Lachnospira, Bilophila, Ruminococcus,* and *Roseburia* are at different concentrations in patients with MS compared to the general population and this data may be used as a diagnostic test to more preferably be used together with tests already existing for the diagnosis of this pathology. Secondly, it can also be concluded that patients undergoing treatment with a worse clinical progression and worse response to treatment exhibit microbiota different from the microbiota of patients with MS who do respond to treatment, specifically and in this case they exhibit differences in the levels of at least the bacteria *belonging to the genus Ezakiella.*

It must be noted that in the context of the present invention, the genus ***Roseburia*** is found at lower levels in biological intestinal or stool samples in patients with MS compared to the general population. Furthermore, it must be noted that the following sequences SEQ ID NO 1 to SEQ ID NO 3 are sequences representative of this genus and are therefore useful for the identification of bacteria belonging to this genus in said samples through a PCR method or by means of any sequencing technique.

## SEQ ID NO 1>>bcfaebf36a7617af86ae9f48670ad25b

```
GGGAATATTGCACAATGGGGGAAACCCTGATGCAGCGACGCCGCGTGAGCG
AAGAAGTATTTCGGTATGTAAAGCTCTATCAGCAGGGAAGAAGAATGACGGT
ACCTGACTAAGAAGCACCGGCTAAATACGTGCCAGCAGCCGCGGTAATACGT
ATGGTGCAAGCGTTATCCGGATTTACTGGGTGTAAAGGGAGCGCAGGCGGTA
CGGCAAGTCTGATGTGAAATCCCGGGGCTCAACCCCGGTACTGCATTGGAAA
CTGTCGGACTAGAGTGTCGGAGGGGTAAGTGGAATTCCTAGTGTAGCGGTGA
AATGCGTAGATATTAGGAGGAACACCAGTGGCGAAGGCGGCTTACTGGACGA
TTACTGACGCTGAGGCTCGAAAGCGTGGGGAGCAAAC
```

SEQ ID NO 2 >62ea8104bf71ebaa6f22b6a761e62854

```
GGGAATATTGCACAATGGGGGAAACCCTGATGCAGCGACGCCGCGTGAGCG
AAGAAGTATTTCGGTATGTAAAGCTCTATCAGCAGGGAAGAAAAAATGACG
GTACCTGACTAAGAAGCCCCGGCTAACTACGTGCCAGCAGCCGCGGTAATAC
GTAGGGGGCAAGCGTTATCCGGATTTACTGGGTGTAAAGGGAGCGCAGGCGG
TGCGGCAAGTCTGATGTGAAAGCCCGGGGCTCAACCCCGGGACTGCATTGGA
AACTGTCGTACTTGAGTATCGGAGAGGTAAGTGGAATTCCTAGTGTAGCGGT
GAAATGCGTAGATATTAGGAGGAACACCAGTGGCGAAGGCGGCTTACTGGAC
GATAACTGACGCTGAGGCTCGAAAGCGTGGGGAGCAAAC
```

SEQ ID NO 3>>62df74476957d3c919c8c93fb44ef348

GGGAATATTGCACAATGGGGGAAACCCTGATGCAGCGACGCCGCGTGAGCG
AAGAAGTATTTCGGTATGTAAAGCTCTATCAGCAGGGAAGAAGAAATGACGG
TACCTGACTAAGAAGCACCGGCTAAATACGTGCCAGCAGCCGCGGTAATACG
TATGGTGCAAGCGTTATCCGGATTTACTGGGTGTAAAGGGAGCGCAGGCGGA
AGGCTAAGTCTGATGTGAAAGCCCGGGGCTCAACCCCGGTACTGCATTGGAA
ACTGGTCATCTAGAGTGTCGGAGGGGTAAGTGGAATTCCTAGTGTAGCGGTG
AAATGCGTAGATATTAGGAGGAACACCAGTGGCGAAGGCGGCTTACTGGACG
ATAACTGACGCTGAGGCTCGAAAGCGTGGGGAGCAAAC

One skilled in the art will have knowledge of other sequences representative of this genus which will be used for identifying those bacteria belonging to this genus in a stool or intestinal sample.

Moreover, it must be noted that in the context of the present invention the genus *Lachnospira* is found at lower levels in biological intestinal or stool samples in patients with MS compared to the general population. Furthermore, it must be noted that the following sequences SEQ ID NO 4 to SEQ ID NO 6 are sequences representative of this genus and are therefore useful for the identification of bacteria belonging to this genus in said samples through a PCR method or by means of any sequencing technique.

SEQ ID NO 4 >a3723443610f87b1804c73e2aa29abb0

GGGAATATTGCACAATGGGGGAAACCCTGATGCAGCGACGCCGCGTGAGCG
AAGAAGTATTTCGGTATGTAAAGCTCTATCAGCAGGGAAGAAAATGACGGTA

CCTGACTAAGAAGCCCCGGCTAACTACGTGCCAGCAGCCGCGGTAATACGTA
GGGGGCAAGCGTTATCCGGATTTACTGGGTGTAAAGGGAGCGTAGACGGCGA
GGCAAGTCTGATGTGAAAACCCGGGGCTCAACCCCGTGACTGCATTGGAAAC
TGTTTTGCTTGAGTGCCGGAGAGGTAAGCGGAATTCCTAGTGTAGCGGTGAA
ATGCGTAGATATTAGGAGGAACACCAGTGGCGAAGGCGGCTTACTGGACGGC
AACTGACGTTGAGGCTCGAAAGCGTGGGGAGCAAAC

SEQ ID NO 5 >9440de0d588a51ea934e136883c61aac

GGGAATATTGCACAATGGGGGAAACCCTGATGCAGCGACGCCGCGTGAGCG
AAGAAGTATTTCGGTATGTAAAGCTCTATCAGCAGGGAAGAAAATGACGGTA
CCTGAATAAGAAGCCCCGGCTAACTACGTGCCAGCAGCCGCGGTAATACGTA
GGGGGCAAGCGTTATCCGGATTTACTGGGTGTAAAGGGAGCGTAGACGGCGA
GGCAAGTCTGATGTGAAAACCCGGGGCTCAACCCCGTGACTGCATTGGAAAC
TGTTTTGCTTGAGTGCCGGAGAGGTAAGCGGAATTCCTAGTGTAGCGGTGAA
ATGCGTAGATATTAGGAGGAACACCAGTGGCGAAGGCGGCTTACTGGACGGC
AACTGACGTTGAGGCTCGAAAGCGTGGGGAGCAAAC

SEQ ID NO 6 >ea80a53b5eda714de0e6f1f533dcdbdd

```
GGGAATATTGCACAATGGGGGAAACCCTGATGCAGCGACGCCGCGTGAGCG
AAGAAGTATTTCGGTATGTAAAGCTCTATCAGCAGGGAAGAAAATGACGGTA
CCTGAATAAGAAGCCCCGGCTAACTACGTGCCAGCAGCCGCGGTAATACGTA
GGGGGCAAGCGTTATCCGGATTTACTGGGTGTAAAGGGAGCGTAGACGGCGA
GGCAAGTCTGATGTGAAAGCCCGGGGCTCAACCCCGTGACTGCATTGGAAAC
TGTTTTGCTTGAGTGCCGGAGAGGTAAGCGGAATTCCTAGTGTAGCGGTGAA
ATGCGTAGATATTAGGAGGAACACCAGTGGCGAAGGCGGCTTACTGGACGGC
AACTGACGTTGAGGCTCGAAAGCGTGGGGAGCAAAC
```

Furthermore, it must be noted that in the context of the present invention, the genus *Ezakiella* is found at higher levels in biological intestinal or stool samples in patients with MS with respect to the general population. Furthermore, it must be noted that the following sequences SEQ ID NO 7 to SEQ ID NO 9 are sequences representative of this genus and are therefore useful for the identification of bacteria belonging to this genus in said samples through a PCR method or by means of any sequencing technique.

SEQ ID NO 7 >b90dde667311a3c4c3d8bc49cded2d69

```
GGGAATATTGCACAATGGGGGAAACCCTGATGCAGCGACGCCGCGTGAGCGATGA
AGGCTTTCGAGTCGTAAAGCTCTGTCCTATGGGAAGATAATGACGGTACCATAGAA
GAAAGCCCCGGCTAATTACGTGCCAGCAGCCGCGGTAATACGTAAGGGGCGAGCG
TTGTCCGGAATTATTGGGCGTAAAGAGTGCGTAGGCGGCAAATTAAGTCAGATGTG
AAAACTAAGGGCTCAACCCATAGATTGCATCTGAAACTGATATGCTTGAGTCAAGG
AGAGGAAAGTGGAATTCCTAGTGTAGCGGTGGAATGCGTAGATATTAGGAGGAAT
ACCGGTGGCGAAGGCGACTTTCTGGACTTGAACTGACGCTGAGGCACGAAAGCGTG
GGGAGCAAAC
```

SEQ ID NO 8 >670e10e4a4802959dbb90a7dbc991f22

```
GGGAATATTGCACAATGGAGGGAACTCTGATGCAGCGACGCCGCGTGAGTGATGA
AGGAATTCGTTTCGTAAAACTCTGTTCTATGGGAAGAAAAGGACTGTACCATAGGA
GAAAGCTCCGGCTAAATACGTGCCAGCAGCCGCGGTAATACGTATGGAGCGAGCG
TTGTCCGGAATTATTGGGCGTAAAGGGTGCGCAGGCGGCTTTACAAGTTGGATGTG
AAATATTGTGGCTCAACCACAAACGTGCATCCAAAACTGCAAAGCTTGAGTTAAGG
AGAGGTAAGTGGAATTCCTGGTGTAGCGGTGGAATGCGTAGATATCAGGAGGAAT
ACCGGTGGCGAAGGCGACTTACTGGACTTAAACTGACGCTCAGGCACGAAAGCGT
GGGGAGCAAAC
```

SEQ ID NO 9 >737f245bd31abba4f81243d008fd9e34

```
GGGAATATTGCACAATGGAGGGAACTCTGATGCAGCGACGCCGCGTGAGTGATGA
AGGAATTCGTTTCGTAAAACTCTGTTCTATGGGAAGAAAAGGACTGTACCATAGGA
GAAAGCTCCGGCTAAATACGTGCCAGCAGCCGCGGTAATACGTATGGAGCGAGCG
TTGTCCGGAATTATTGGGCGTAAAGGGTGCGCAGGCGGCTTTACAAGTTGGATGTG
AAATATTGTGGCTCAACCACAAACGTGCATCCAAAACTGCAAAGCTTGAGTTAAGG
AGAGGTAAGTGGAATTCCTGGTGTAGCGGTGGAATGCGTAGATATCAGGAGGAAT
ACCGGTGGCGAAGGCGACTTACTGGACTTAAACTGACGCTCAGGCACGAAAGCGT
GGGGAGCAAACA
```

One skilled in the art will have knowledge of other sequences representative of this genus which will be used for identifying those bacteria belonging to this genus in a stool or intestinal sample.

Finally, it must be noted that in the context of the present invention, the genus *Bilophila* is found at higher levels in biological intestinal or stool samples in patients with MS with respect to the general population. Furthermore, it must be noted that the following sequences SEQ ID NO 10 to SEQ ID NO 12 are sequences representative of this genus and are therefore useful for the identification of bacteria belonging to this genus in said samples through a PCR method or by means of any sequencing technique.


SEQ ID NO 10 >8f8744e700fb76316b7af104f71ac34e

GGGAATATTGCGCAATGGGCGAAAGCCTGACGCAGCGACGCCGCGTGAGGGATGA
AGGTTCTCGGATCGTAAACCTCTGTCAGGGGGGAAGAAACCCCCTCGTGTGAATAA
TGCGAGGGCTTGACGGTACCCCCAAAGGAAGCACCGGCTAACTCCGTGCCAGCAGC
CGCGGTAATACGGAGGGTGCAAGCGTTAATCGGAATCACTGGGCGTAAAGCGCAC
GTAGGCGGCTTGGTAAGTCAGGGGTGAAATCCCACAGCCCAACTGTGGAACTGCCT
TTGATACTGCCAGGCTTGAGTACCGGAGAGGGTGGCGGAATTCCAGGTGTAGGAGT
GAAATCCGTAGATATCTGGAGGAACACCGGTGGCGAAGGCGGCCACCTGGACGGT
AACTGACGCTGAGGTGCGAAAGCGTGGGTAGCAAAC


SEQ ID NO 11 >dad0fb8c339eb1499fb4e4d1e6b974c2

GGGAATATTGCGCAATGGGCGAAAGCCTGACGCAGCGACGCCGCGTGAGGGATGA
AGGTTCTCGGATCGTAAACCCCTGTCAGGGGGGAAGAAACCCCCTCGTGTGAATAA
TGCGAGGGCTTGACGGTACCCCCAAAGGAAGCACCGGCTAACTCCGTGCCAGCAGC
CGCGGTAATACGGAGGGTGCAAGCGTTAATCGGAATCACTGGGCGTAAAGCGCAC
GTAGGCGGCTTGGTAAGTCAGGGGTGAAATCCCACAGCCCAACTGTGGAACTGCCT
TTGATACTGCCAGGCTTGAGTACCGGAGAGGGTGGCGGAATTCCAGGTGTAGGAGT
GAAATCCGTAGATATCTGGAGGAACACCGGTGGCGAAGGCGGCCACCTGGACGGT
AACTGACGCTGAGGTGCGAAAGCGTGGGTAGCAAAC


SEQ ID NO 12 >fca339d0dd6b1dec5d76f2a96dfe0182

GGGAATATTGCGCAATGGGCGAAAGCCTGACGCAGCGACGCCGCGTGAGGGATGA
AGGTTCTCGGATCGTAAACCTCTGTCAGGGGGGAAGAAACCCCCTCGTGTGAATAA
TGCGAGGGCTTGACGGTACCCCCAAAGGAAGCACCGGCTAACTCCGTGCCAGCAGC
CGCGGTAATACGGAGGGTGCAAGCGTTAATCGGAATCACTGGGCGTAAAGCGCAC
GTAGGCGGCTTGGTAAGTCAGGGGTGAAATCCCACAGCCCAACTGTGGAACTGCCT
TTGATACTGTCAGGCTTGAGTACCGGAGAGGGTGGCGGAATTCCAGGTGTAGGAGT
GAAATCCGTAGATATCTGGAGGAACACCGGTGGCGAAGGCGGCCACCTGGACGGT
AACTGACGCTGAGGTGCGAAAGCGTGGGTAGCAAAC


One skilled in the art will have knowledge of other sequences representative of this genus which will be used for identifying those bacteria belonging to this genus in a stool or intestinal sample. It must be noted that in the context of the present invention, the genus *Ruminococcus* is found at higher levels in biological intestinal or stool samples in patients with MS compared to the general population. Furthermore, it must be noted that the following sequences SEQ ID NO 13 to SEQ ID NO 15 are sequences representative of this genus and are therefore useful for the identification of bacteria belonging to this genus in said samples through a PCR method or by means of any sequencing technique.

SEQ ID NO 13 >ca7d260498fc98f5b1203d3a5bf0cba0

GGGAATATTGCGCAATGGAGGAAACTCTGACGCAGTGACGCCGCGTATAGGAAGA
AGGTTTTCGGATTGTAAACTATTGTCGTTAGGGAAGATACAAGACAGTACCTAAGG
AGGAAGCTCCGGCTAACTACGTGCCAGCAGCCGCGGTAATACGTAGGGAGCAAGC
GTTATCCGGATTTATTGGGTGTAAAGGGTGCGTAGACGGGACAACAAGTTAGTTGT
GAAATCCCTCGGCTTAACTGAGGAACTGCAACTAAAACTATTGTTCTTGAGTGTTG
GAGAGGAAAGTGGAATTCCTAGTGTAGCGGTGAAATGCGTAGATATTAGGAGGAA
CACCGGTGGCGAAGGCGACTTTCTGGACAATAACTGACGTTGAGGCACGAAAGTGT
GGGGAGCAAAC

SEQ ID NO 14>4142cb390a45ceac09f3fef2846b7d22

GGGAATATTGGACAATGGAGGAAACTCTGATCCAGTGACGCCGCGTGAAGGAAGA
AGGTCTTCGGATTGTAAACTTATTTTATCAGGGAAGAAGAAAGTGACAGTACCTGA
AGAAAAAGGACCGGCAAACTACGTGCCAGCAGCCGCGGTAATACGTAGGGTCCAA
GCGTTATCCGGATTTACTGGGTGTAAAGGGCGAGTAGACGGCGCTGTAAGTCAGCT
GTGAAAACTTAGGGCTCAACCTTAAGCCTGCAGCTGAAACTGTAGTGCTAGAGTGC
AGGAGAGGTAAGCGGAATTCCTAGTGTAGCGGTGAAATGCGTAGATATTAGGAGG
AACACCAGTGGCGAAGGCGGCTTACTGGACTGTAACTGACGTTGAGGCGCGAAAG
TGTGGGGAGCAAAC

SEQ ID NO 15>471de9965b88e25338c306b99ecede17

GGGAATATTGCGCAATGGAGGAAACTCTGACGCAGTGACGCCGCGTGCAGGAAGA
AGGTTTTCGGATTGTAAACTGCTTTTAACAGGAAAGAAAAAAATGACGGTACCTGT
TGAATAAGCTCCGGCTAACTACGTGCCAGCAGCCGCGGTAATACGTAGGGAGCGA
GCGTTATCCGGATTTATTGGGTGTAAAGGGTGCGTAGACGGAAATGCAAGTTAGTT
GTGAAATACCTCGGCTTAACTGAGGAACTGCAACTAAAACTATATTTCTTGAGTAT
CGGAGGGGTTTGTGGAATTCCTAGTGTAGCGGTGAAATGCGTAGATATTAGGAAGA

ACACCGGTGGCGAAGGCGACAAACTGGACGATAACTGACGTTGAGGCACGAAAGT
GTGGGGAGCAAAC

One skilled in the art will have knowledge of other sequences representative of this genus which will be used for identifying those bacteria belonging to this genus in a stool or intestinal sample. The following Table 1 summarizes the **diagnostic** information indicated above.

Table 1

| Genus | Correlation | Value | Sensitivity | Specificity |
|---|---|---|---|---|
| *Roseburia* | - | 390 | 0.92 | 0.63 |
| *Lachnospira* | - | 120 | 0.35 | 0.86 |
| *Ezakiella* | + | 2.5 | 1 | 0.57 |
| *Bilophila* | + | 52 | 0.71 | 0.85 |
| *Ruminococcus* | + | 22 | 0.86 | 0.65 |

Therefore, a first aspect of the present invention relates to the *in vitro* use of the level or the concentration in an intestinal or stool sample of bacteria belonging to the genus *Ezakiella, Lachnospira, Bilophila, Ruminococcus,* or *Roseburia,* or any combination thereof, for the diagnosis/of multiple sclerosis in a patient, or for obtaining useful data which allows said diagnosis.

According to the data shown in the examples, for diagnosis, *Roseburia* with a 390 cut-off point, 92% sensitivity, and 63% specificity would be the best indicator of the presence of MS individually. If all the indicators (*Roseburia, Ezakiella, Bilophila,* and *Lachnospira,* and optionally *Ruminococcus*) were combined, sensitivity would increase to 100% and *Ezakiella* with a 2.5 cut-off point (57% specificity and 100% sensitivity), *Bilophila* with a 52 cut-off point (85% specificity and 71% sensitivity), and *Lachnospira* with a 120 cut-off point (86% specificity and 35% sensitivity) could be used.

An alternative embodiment of the first aspect of the invention relates to a method for the *in vitro* diagnosis or for obtaining useful data which helps in said diagnosis of a subject suspected of possibly suffering from multiple sclerosis, which method comprises using, as an indicator in an intestinal or stool sample obtained from said subject, the level or the concentration in said sample of bacteria belonging to the genus *Ezakiella, Lachnospira, Bilophila, Ruminococcus,* or *Roseburia,* or any combination thereof, wherein if the level or the concentration in said intestinal or stool sample of bacteria belonging to *Ezakiella, Lachnospira, Bilophila, Ruminococcus,* or *Roseburia,* or any combination thereof differs or varies from that present in an intestinal or stool sample obtained from a healthy subject or with respect to a reference value, it is indicative of said subject having multiple sclerosis.

In a preferred embodiment of the first aspect of the invention, the method comprises using, as an indicator in an intestinal or stool sample obtained from said subject, the level or the concentration in said sample of bacteria belonging to the genus *Bilophila,* wherein if the level or the concentration in said intestinal or stool sample of bacteria belonging to said genus is increased with respect to that present in an intestinal or stool sample obtained from a healthy subject or with respect to a reference value, it is indicative of said subject having multiple sclerosis.

In another preferred embodiment of the first aspect of the invention, the method comprises using, as an indicator in an intestinal or stool sample obtained from said subject, of the level or the concentration in said sample of bacteria belonging to the genus *Ruminococcus,* wherein if the level or the concentration in said intestinal or stool sample of bacteria belonging to said genus is increased with respect to that present in an intestinal or stool sample obtained from a healthy subject or with respect to a reference value, it is indicative of said subject having multiple sclerosis.

In another preferred embodiment of the first aspect of the invention, the method comprises using, as an indicator in an intestinal or stool sample obtained from said subject, the level or the concentration in said sample of bacteria belonging to the genus *Ezakiella,* wherein if the level or the concentration in said intestinal or stool sample of bacteria belonging to said genus is increased with respect to that present in an intestinal or stool sample obtained from a healthy subject or with respect to a reference value, it is indicative of said subject having multiple sclerosis.

In another preferred embodiment of the first aspect of the invention, the method comprises using, as an indicator in an intestinal or stool sample obtained from said subject, the level or the concentration in said sample of bacteria belonging to the genus *Lachnospira,* wherein if the level or the concentration in said intestinal or stool sample of bacteria belonging to said genus is decreased with respect to that present in an intestinal or stool sample obtained from a healthy subject or with respect to a reference value, it is indicative of said subject having multiple sclerosis.

In another preferred embodiment of the first aspect of the invention, the method comprises using, as an indicator in an intestinal or stool sample obtained from said subject, the level or the concentration in said sample of bacteria belonging to the genus *Roseburia,* wherein if the level or the concentration in said intestinal or stool sample of bacteria belonging to said genus is decreased with respect to that present in an intestinal or stool sample obtained from a healthy subject or with respect to a reference value, it is indicative of said subject having multiple sclerosis.

In yet another preferred embodiment of the first aspect of the invention, the method comprises using, as an indicator in an intestinal or stool sample obtained from said subject, the level or the concentration in said sample of bacteria belonging to the genera *Ezakiella, Lachnospira, Bilophila,* and *Roseburia, and* optionally *Ruminococcus,* wherein if the level or the concentration in said intestinal or stool sample of bacteria belonging to the genera *Roseburia* and *Lachnospira* is decreased with respect to that present in an intestinal or stool sample obtained from a healthy subject or with respect to a reference value, and wherein if the level or the concentration in said intestinal or stool sample of bacteria belonging to the genera *Ezakiella, Ruminococcus,* and *Bilophila* is increased with respect to that present in an intestinal or stool sample obtained from a healthy subject or with respect to a reference value, it is indicative of said subject having multiple sclerosis.

In the context of the present invention, multiple sclerosis is understood to be a progressive disease of the central nervous system causing multiple lesions in the myelin covering axons of neurons and constituting the white substance, in the form of disseminated plaques; and RRMS is understood to be a predominant type of multiple sclerosis in which symptoms present in the form of flare-ups that can last for days, weeks, and even months, and will vary from one episode to another, according to the affected area of the central nervous system.

It must be noted that the present invention sufficiently describes the representative nucleotide sequences (i.e., SEQ ID No 1 to SEQ No 15) which allow identifying the presence as well as the concentration of each of the genera herein mentioned.

A second aspect of the present invention relates to an *in vitro* method for the prognosis of the progression of multiple sclerosis in a subject, comprising the following steps:

a. determining the levels or the concentration of bacteria belonging to the genus *Ezakiella* in an intestinal or stool sample isolated from said subject; and

b. comparing the levels or the concentration of said bacteria in said intestinal or stool sample with the value obtained in a sample from a healthy subject or with a reference value, wherein for every 100 units of increase in the sequences of *Ezakiella* in said sample with respect to the control, the increase in the risk of flare-ups goes up preferably by an approximate value of 1.56, in comparison with patients with zero flare-ups. In other words, an increase in the level or the concentration of bacteria belonging to the genus *Ezakiella* in an intestinal or stool sample isolated from said subject with respect to the control is indicative of a poor progression of the disease.

In the context of the present invention, poor progression of the disease is understood to be the presence of one or more new flare-ups of the disease regardless of whether or not the multiple sclerosis is being treated. However, preferably in the context of the present invention said prognosis is carried out in subjects treated with any of the drugs prescribed for this disease, preferably with one or more of those drugs identified in Table 2.

In the context of the present invention, reference value is preferably understood to be the result of the data of a mathematical algorithm which uses the concentration and the total amount of each bacterium belonging to one or more of the genera proposed in the present invention in the general population or in a healthy subject. The best sensitivity and specificity value will automatically be proposed using the algorithm. This algorithm will provide values along with the proposed value, with the changing sensitivity and specificity of the values in order to provide physicians with more information and to allow them to decide on the best test and cut-off value for each patient or specific situation.

In the context of the present invention, the bacteria belonging to any of the genera proposed in an intestinal or stool sample isolated from said subject can be determined, in a non-limiting manner, by means of massive sequencing of the genome of the stool such that the total number of sequences of that bacterium existing in the stool is obtained together with the total number of other bacteria present in that stool sample. Preferably, said determination is performed by means of a PCR or real-time PCR method.

In another preferred embodiment of the first or second aspect of the invention or of any of the preferred embodiments of the invention, said levels or concentration of bacteria refer to the total amount of the bacteria belonging to the genus with respect to the total bacteria present in said sample.

In another preferred embodiment of the first or second aspect of the invention or of any of the preferred embodiments of the invention, the multiple sclerosis is relapsing-remitting multiple sclerosis (RRMS).

In another preferred embodiment of the first or second aspect of the invention or of any of the preferred embodiments of the invention, the levels or the concentration of bacteria belonging to the genera *Ezakiella, Lachnospira, Bilophila,* and/or *Roseburia* are determined by performing an amplification reaction from a nucleic acid preparation derived from said sample using a pair of primers capable of amplifying one or more representative regions of said genera.

In yet another preferred embodiment of the first or second aspect of the invention or of any of the preferred embodiments of the invention, the amplification reaction is carried out by means of a real-time polymerase chain reaction. Preferably, the detection of the amplification product is carried out by means of a fluorescent intercalating agent. Even more preferably, the detection of the amplification product(s) is carried out by means of a labeled probe, wherein the probe preferably comprises at its 5' end a reporter pigment and at its 3' end a "quencher" pigment or silencer or buffering agent.

A third aspect of the invention relates to an *in vitro* method for the prediction of the therapeutic response of a patient diagnosed with multiple sclerosis, preferably relapsing-remitting multiple sclerosis, wherein said method comprises the steps of determining and comparing the levels or the concentration of bacteria according to any of the embodiments of the second aspect of the invention, and wherein an increase in the level or the concentration of bacteria belonging to the genus *Ezakiella* is indicative of a lack of response to treatment, preferably of any of the treatments identified in Table 2.

In a fourth aspect of the invention, the method of the first, second, or third aspect of the invention or of any of the embodiments thereof further comprises storing the results of the method in a data carrier, wherein said data carrier is preferably a computer readable medium.

In a fifth aspect of the invention, the method of the first, second, or third aspect of the invention or of any of the embodiments thereof comprises at least the implementation of the comparative step and optionally the provision of a result as a consequence of said comparison using a computer program.

A sixth aspect of the invention relates to a kit comprising one or more pairs of primers capable of amplifying bacteria belonging to the genera *Ezakiella, Lachnospira, Bilophila,* and/or *Roseburia*. Preferably and as mentioned, a sixth aspect of the invention relates to a method for the detection of bacteria from a stool or intestinal sample, comprising the following steps:

> i) contacting the sample to be analyzed with a reaction mixture containing specific primers capable of amplifying bacteria belonging to the genera *Ezakiella, Lachnospira, Bilophila,* and/or *Roseburia,* preferably for performing multiplex PCR,
> ii) performing amplification by means of polymerase chain reaction,
> iii) identifying the formation of the products of the preceding step, said formation being indicative of the levels or the concentration of bacteria belonging to one or more of the genera *Ezakiella, Lachnospira, Bilophila,* and/or *Roseburia.*

In relation to this sixth aspect of the invention, it preferably provides a method for simultaneously detecting *Ezakiella, Lachnospira, Bilophila,* and/or *Roseburia.*

In a particular embodiment of this sixth aspect of the invention, DNA fragments included or comprised in sequences 1 to 12 are amplified.

In another embodiment of this sixth aspect of the invention, the amplification products which allow identifying the different bacterial species and groups are detected by means of using probes. In a more preferred embodiment, these probes have a length between 15 and 25 nucleotides. The primers can be designed by means of multiple alignment with programs such as CLUSTAL X, which allow identifying highly conserved regions that serve as a template. In another particular embodiment of this first

Given the great abundance of PCR inhibitors, such as humic and fulvic acids, heavy metals, heparin, etc., which may give rise to false negatives, and despite the existence of methods which reduce the concentration of molecules of this type, it is advisable (cf. J. Hoorfar et al., "Making internal amplification control mandatory for diagnostic PCR" J. of Clinical Microbiology, Dec. 2003, pp. 5835) for PCR assays to contain an internal amplification control (IAC). This IAC is simply a DNA fragment which is amplified simultaneously with the target sample, such that its absence at the end of the assays is indicative of the presence of factors which have led to an unwanted PCR development.

Throughout the description, the term "specific" means that the primers comprise a nucleotide sequence that is completely complementary to the genes or gene fragments used by the present invention.

A preferred embodiment of the sixth aspect of the invention relates to the use of the kit or of the methodology therein described for implementing the methodology according to any of the first, second, or third aspects of the present invention.

In summary, the knowledge acquired about gut microbiota composition in patients undergoing treatment along with the development of the diagnosis or prognosis KIT based on this gut microbiota is of crucial importance, as it would allow the neurologist treating these patients to attain data about the probability of the patient presenting or not presenting multiple sclerosis during their first visits, as well as the probability of response to treatment by identifying those patients with a worse prognosis.

The following examples merely illustrate the present invention and must not be understood as limiting same.

**Examples of the Invention:**

**Example 1: Definition of criteria for the inclusion and selection of patients with relapsing-remitting MS:**

MATERIALS AND METHODS

*Characteristics of the population under study*

Patients evaluated for inclusion in the study had been previously diagnosed with RRMS according to clinical and radiological findings defined by the presence of at least one flare-up (concept clinically described as the onset of new symptoms or the worsening of a previous symptom, with a duration greater than 24 hours and less than 3 months after a period of stability of at least one month) or new damage to the CNS, documented by means of magnetic resonance imaging in the year prior to inclusion.

The participants provided socio-demographic information and clinical data including age, sex, pharmacological treatment, and the regular diet they followed over the past year. Furthermore, the BMI (body mass index) of all participants was obtained. All the patients submitted a stool sample which was collected in sterile conditions and immediately frozen at -80°C until its subsequent processing for genome sequencing. The exclusion criteria were as follows: use of immunosuppressants such as systemic corticoids, methotrexate, cyclosporine, or tumor necrosis factor alpha (TNF$\alpha$) prescribed for comorbidity in the three months prior, systemic antibiotics in the two weeks prior, and the concomitant diagnosis of cirrhosis, inflammatory bowel disease, and signs of bacterial infection.

The study was approved by the Research Committee of the hospital center in which the research was conducted in accordance with the protocol of ethical principles for medical research involving human subjects of the Declaration of Helsinki. All the patients were given the informed consent document which they signed and returned prior to their inclusion in the study.

RESULTS

From May to June 2016, 16 patients diagnosed with RRMS participated in the study. Fifteen (93.75%) patients were women with a mean age of 38.15 ± 8.08. Thirteen (81.25%) patients were undergoing treatment for MS when they were included in the study, with interferon beta (5 out of 16; 31.3%) and fingolimod (3 out of 16; 18.8%) being the most frequently administered medication. All the patients exhibited activity of the disease in the year prior to inclusion, with myelitis being the most prevalent type of presentation (6 out of 16; 37.5%). The baseline characteristics of the patients are described in Table 2.

**Table 2.** Baseline demography and clinical data of the patients

| Patient | Gender | Age (years) | Weight (kg) | Height | BMI (kg/m²) | Treatment | Clinical presentation | Flare-ups | CNS lesions |
|---|---|---|---|---|---|---|---|---|---|
| 1 | Female | 38 | 90 | 1.72 | 30.42 | Interferon beta 1a* | Infratentorial | 0 | 0 |
| 2 | Female | 18 | 57 | 1.71 | 19.49 | Interferon beta 1a | Hemihypoesthesia | 1 | 1 |
| 3 | Female | 38 | 57 | 1.54 | 22.77 | Natalizumab* | Brainstem | 0 | 0 |
| 4 | Female | 35 | 59 | 1.51 | 25.88 | Fingolimod | Myelitis | 0 | 0 |
| 5 | Female | 35 | 107 | 1.78 | 33.77 | Dimethyl fumarate | Myelitis + Brainstem | 1 | 0 |
| 6 | Female | 40 | 48 | 1.51 | 16.67 | Fingolimod | Myelitis | 1 | 0 |
| 7 | Female | 34 | 75.5 | 1.6 | 29.49 | Interferon beta 1b | Hemiparesis | 3 | 6 |
| 8 | Female | 31 | 100 | 1.8 | 30.86 | Fingolimod* | Hemiparesis | 2 | 5 |
| 9 | Female | 33 | 85.5 | 1.7 | 29.58 | Natalizumab | Brainstem | 0 | 0 |
| 10 | Female | 45 | 59 | 1.65 | 21.67 | Fingolimod | Myelitis | 2 | 8 |
| 11 | Female | 53 | 92.7 | 1.55 | 38.58 | 44 mcg interferon beta 1a | Myelitis | 0 | 2 |
| 12 | Female | 37 | 63 | 1.69 | 22.05 | Interferon beta 1b | Brainstem | 0 | 0 |
| 13 | Male | 44 | 92 | 1.73 | 30.77 | Teriflunomide | Myelitis | 2 | 3 |
| 14 | Female | 29 | 54 | 1.6 | 21.09 | Dimethyl fumarate | Hemiparesis | 0 | 3 |
| 15 | Female | 51 | 63 | 1.66 | 22.86 | 44 mcg interferon beta 1a | Infratentorial + Optic neuritis | 1 | 4 |
| 16 | Female | 37 | 59 | 1.51 | 25.88 | Glatiramer acetate | Brainstem | 0 | 0 |

BMI: Body mass index. Number of flare-ups and CNS lesions during the last 3 years of patient follow-up.

*Undergoing treatment the year prior to inclusion in the study but not undergoing treatment when the stool sample was collected.

**Example 2: Determination of the gut microbiota composition of patients undergoing treatment for relapsing-remitting MS through massive stool sample genome sequencing**

MATERIALS AND METHODS

*Massive stool sample genome sequencing for patients with RRMS*

The DNA of stool samples was isolated with the help of the "MagnaPure Compact" system (Roche LifeScience) to prevent biases in the purification of the Gram positive bacteria DNA, following the method of Yuan *et al.* with minor modifications. In the massive sequencing analysis, the hypervariable region V3-V4 of the 16S rRNA bacterial gene was amplified using labeled eubacterial primers and sequenced with the MiSeqIllumina platform following Illumina's recommendations for library preparation and sequencing in metagenomic studies. The bacterial composition of all the patients was compared with a cohort of healthy individuals matched for sex and age extracted from an internal data set of people.

*Bioinformatics*

The resulting sequences were divided taking into account the barcode introduced during the PCR reaction, whereas readings R1 and R2 were overlapped using the PEAR program version 0.9.122 which provides a single FASTQ file for each of the samples. Sequence quality control was performed in different steps, i) quality filtering (with a minimum threshold of Q20) was performed using the rapid toolkit version 0.013, ii) the primer (16s rRNA primers) the cutting and length selection (reads more than 300 nucleotides) was carried out with the cutadapt version 1.4.123. These FASTQ files were converted into FASTA files and the UCHIME program version 7.0.1001 was used to eliminate the chimeras that may arise during the amplification and sequencing stage. Those clean FASTA files were transferred to NCBI's 16S rRNA database using Blast.24 version 2.2.29+. The resulting XML files were processed using a Python script by the Department of Statistics of Biotech Bioithas (Parc Cientific de la Universitat d'Alacant, Spain) to associate each sequence with different phylogenetic levels (phylum, family, genus, and species).

RESULTS

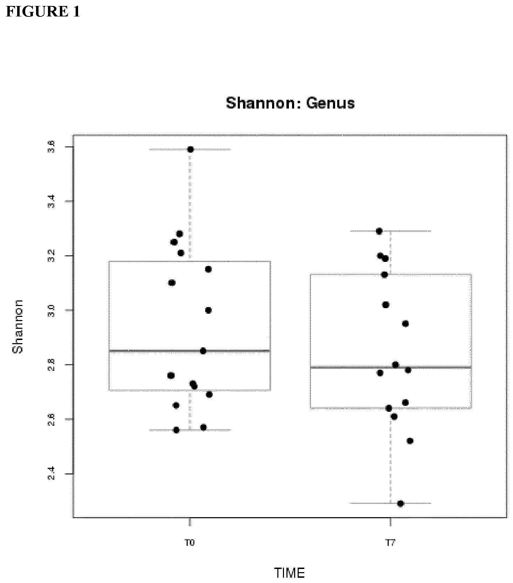

The intestinal bacterial composition in all the patients was analyzed by means of massive genome sequencing. This allowed describing an "RRMS microbiota" based on the mean values of the bacteria detected in all the patients. Analysis at the genus level shows more than 100 different genera in the intestinal microbiota in 15 out of the 16 subjects of the study (one of them did not provide any stool sample for analysis). The genus most commonly detected among the analyzed stool samples was *Bacteroides.* Other common genera were *Roseburia, Desulfovibrio, Erysipelotoclostridium, Gemmiger, Lachnoclostridium, Parabacteroides, Prevotella, Ruminiclostridium, Ruminococcus, Sutterella,* and *Tyzzerella.* The bacterial composition of all the patients with RRMS was compared with the group of healthy volunteers. The Shannon biodiversity index was calculated (mean, (IQR)) in healthy individuals (2.43, (1.80 - 2.57)) and patients with RRMS (3.05 (2.863.31)). Furthermore, significant differences were observed when comparing the means ($p < 0.01$), showing greater variability in the control group. The differences between both groups of subjects appear in the biodiversity of the bacterial composition, which was greater in the healthy group than in the RRMS group (Figure 1). Statistically significant differences were detected between the two population groups in the taxonomic levels of family and genus previously described and included in Figures 2a and 2b.

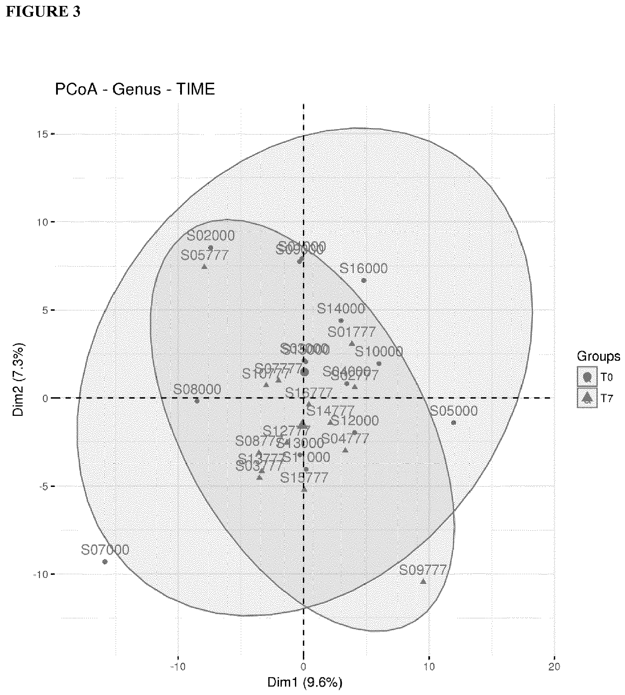

In order to find a possible genus of bacteria that may allow grouping patient data, an analysis of the main components of each of the patients comparing the bacterial composition at the taxonomic genus level was performed (Figure 3).

**Example 3: Statistical analysis of the gut microbiota composition of patients undergoing treatment for RRMS**

MATERIALS AND METHODS

**Statistical analysis**

The quantitative descriptive variables were expressed in terms of mean $\pm$ standard deviation (SD), median, and first and third quartiles. Qualitative variables are indicated as frequencies. In the comparisons between the groups, the U Mann-Whitney test was performed for the quantitative data. The qualitative variables were analyzed with the Chi-square and Fisher tests. The odds ratio (OR) was taken as a measurements of the size of the effect, with a 95% confidence interval (CI). A 2-tailed p-value less than 0.05 was considered statistically significant. All statistical analyses were performed with IBM SPSS statistics version 22 (SPSS Inc. Chicago, IL) and R version 3.2.3. In the microbiome analysis, alpha diversity was estimated using the Specaccum program in the Vegan package implemented for version 3.2.3 of R.

RESULTS

In order to find a possible genus of bacteria which may allow grouping patient data, an analysis of the main components of each of the patients comparing the bacterial composition at the taxonomic genus level was performed (Figure 3).

Finally, all the identified bacteria were subsequently included in a Poisson statistical model to evaluate the possible association of each of these microorganisms with the progression of RRMS based on the number of relapses during the 3 years of the follow-up period. This analysis shows a statistically significant correlation between the number of new relapses and the detected levels of various germs. Furthermore, in the previous analysis it was observed that several of these genera are found in different concentrations in this population when comparing the results with the healthy population. This data is included in the tables below:

• *Area under the curve in the main genera involved with significant differences between cases and controls*

| Entity | Multiplier (95% CI) | P-value | AUC (95% CI) |
|---|---|---|---|
| **g_Lachnospira** | **0.06 (0.01 to 0.35)** | **0.00201** | **67.1 (46.7 to 87.6)** |
| **g_Ezakiella** | **13.08 (2.16 to 79.24)** | **0.00516** | **75.0 (60.6 to 89.4)** |
| **g_Ruminococcaceae** | **12.65 (2.09 to 76.62)** | **0.00578** | **68.1 (47.9 to 88.3)** |
| **g_Roseburia** | **0.12 (0.02 to 0.73)** | **0.02149** | **78.1 (60.2 to 96.0)** |
| g_Bilophila | 5.93 (0.98 to 35.91) | 0.05282 | 70.2 (50.1 to 90.4) |

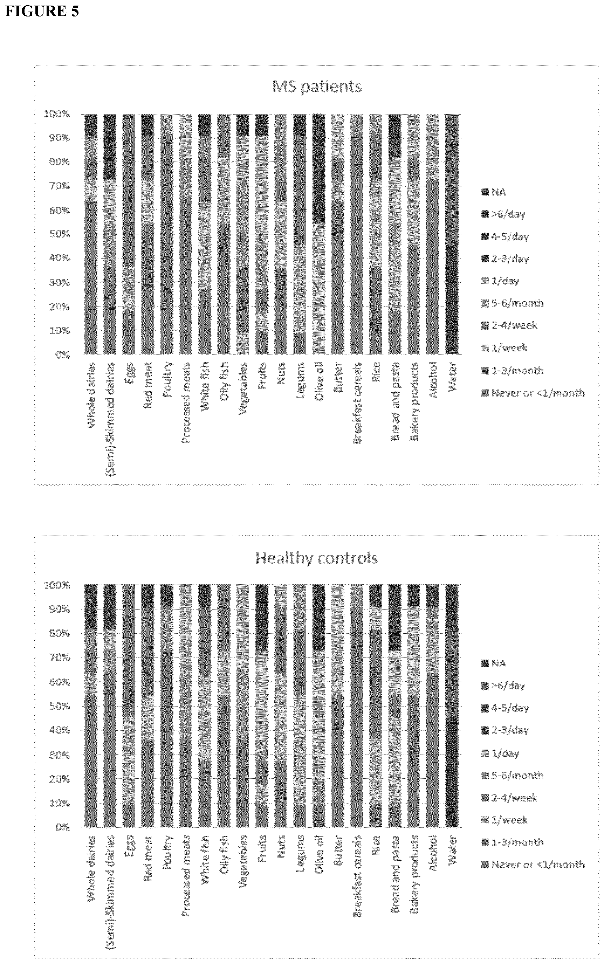

The data concerning the diet and its average components was collected from patients and is shown in Figure 5. The different components of the diet or other variables, such as BMI and age, did not correlate with a worse progression of the disease.

**Example 4: Design of a real-time PCR KIT for the detection of rDNA of microorganisms in the stool of patients with RRMS as a tool for the diagnosis/prognosis or prediction of response of the disease to treatment**

Real-time PCR KIT for the detection of microorganisms in stool.

**Equipment characteristics**

The format used works with individual tubes for each pathogen where it is only necessary to add the sample and perform the PCR. It is a ready-to-use mixture containing all the components required for the detection of specific pathogens: dNTP, polymerase, pathogen-specific priomers and probe, and the appropriate buffer. The sample is simply added and qPCR is performed. It is a ready-to-use dehydrated format. Transport is performed at room temperature, so the enzyme breaking up by freezing/thawing is prevented, and the risk of cross contamination and the deterioration of the fluorophore by UV is minimized.

Format compatible with the following devices: StepOne ™, StepOnePlus ™, ABI 7500 Fast, LightCycler® 96, LightCycler® Nano, CFX96 ™, 24-hole PikoReal ™, DNA Engine® systems, MiniOpticon ™ 48-12, and Opticon® 2. If there is a device that is not included in the list, information about the manufacturer/model must be provided in order to produce MONODOSE in compatible tubes. It must be borne in mind that GPS ™ reagents contain BSA and are compatible with all real-time PCR, glass capillary, or plate-based thermocyclers.

**Identification of bacterial species**

Bacterial identification is based on DNA sequencing of the 16s ribosome (rDNA). The specific sequences of species and genus are obtained from databases and bibliography.

The qPCR equipment contains tubes prepared for use with all the components required for the detection, first and foremost, of the genera *Ezakiella, Lachnospirae, Bilophila, Roseburia,* and optionally *Ruminococcus.*

These microorganisms are an integral part of the fecal flora and none of them has been correlated with a worse progression of the disease or used as a factor for predicting response to treatment.

**Principle of the method**

Polymerase chain reaction (PCR) allows amplifying a concrete and specific region of a DNA strand using specific oligonucleotides. Real-time PCR consists of measuring the amount of amplified product that can be detected in each cycle of thermocycling with fluorescent probes. The increase in signal allows pathogen detection and quantification.

**Contents of the equipment**

The specific probes of the microorganisms included in this equipment are distributed in individual tubes containing dNTPs, BSA, polymerase, and buffer, all of which is dehydrated to stabilize the reagent at room temperature. Moreover, it contains an internal control.
For microorganism quantification, it incorporates a known amount of copies (calibrator) for use as a positive control and for preparing the microorganism quantification curve. It incorporates a buffer for resuspending this calibrator.

**Storage conditions**

All the components of the equipment are stable at room temperature for transport, but they have to be stored at -20°C if not used immediately. The equipment with these characteristics is stable throughout one year of storage (see the expiration date on the label).

**Required material that is not supplied**

- DNA extraction equipment
- DNase-/RNase-free water
- Sterile pipette tips with filter
- Plates for qPCR
- Micropipettes
- Vortex to mix
- Spinner centrifuge
- Ice pack
- Equipment for performing real-time PCR. Format compatible with the following devices: StepOne ™, StepOnePlus ™, ABI 7500 Fast, LightCycler® 96, LightCycler® Nano, CFX96 ™, 24-hole PikoReal ™, DNA Engine® systems, MiniOpticon ™ 48-12, and Opticon® 2

**Preparation of the calibration curve**

1) Pulse-spin the calibrator (red cap), reconstitute with 120 $\mu$l of standard buffer (black cap) and shake, mark as number 1
2) Pipette 900 $\mu$l of DNase-/RNase-free water (not supplied) into 5 tubes marked as numbers 2 to 6
3) Pipette 100 $\mu$l of the calibrator (red cap) already diluted in 120 $\mu$l, and introduce it in tube number 2
4) Shake and perform pulse-spin
5) Change the pipette tip and pipette 100 $\mu$l from tube number 2 to tube number 3
6) Shake and perform pulse-spin
7) Repeat steps 5 and 6 with tubes number 4 to 6 to complete the dilution series.

| Standard curve | copies/$\mu$L | copies in 5 $\mu$L |
|---|---|---|
| Calibrator (red cap) | $2 \times 10^5$ | $1 \times 10^6$ |
| Tube 2 | $2 \times 10^4$ | $1 \times 10^5$ |
| Tube 3 | $2 \times 10^3$ | $1 \times 10^4$ |
| Tube 4 | $2 \times 10^2$ | $1 \times 10^3$ |
| Tube 5 | $2 \times 10$ | $1 \times 10^2$ |
| Tube 6 | 2 | 10 |

Pipette 5 $\mu$l of the calibrator dilutions (tube number 4: 2 x $10^2$ copies/$\mu$L) into each well for preparing the calibration curve. Complete with a final volume of 20 $\mu$l of DNase-/RNase-free water in each reaction well for qPCR.

**Working protocol**

In each well of the qPCR, add 5 $\mu$l of extracted sample and add 15 $\mu$l of DNase-/RNase-free water. Shake and perform pulse-spin.

| Reaction tube | Volume ($\mu$L) |
|---|---|
| Sample | 5 |
| DNase-/RNase-free water | 15 |
| **Final reaction volume** | **20** |

Introduce the samples in the equipment (StepOne ™, StepOnePlus ™, ABI 7500 Fast, LightCycler® 96, Light-Cycler® Nano, CFX96 ™, 24-hole PikoReal ™, DNA Engine® systems, MiniOpticon ™ 48-12, and Opticon® 2) and select the following working protocol:

| qPCR protocol (40 cycles) | Time | Temperature |
|---|---|---|
| Activation | 1 min | 95°C |
| Denaturation | 10 sec | 95°C |
| Hybridization/extension | 1 min | 60°C |

**Recommended reaction controls**

These qPCR reaction controls are recommended considering the ISO/IEC 17025 standardization guidelines.
Negative control (- ctrl): Add DNase-/RNase-free water to a reaction tube. According to this, the reaction should be negative. A positive result must be considered as contamination of the water used in the reaction, so the test must be considered null and void and the water would have to be replaced.
Positive control (+ ctrl): Prepare a calibrator of the dilution curve as a sample, example tube 4, and add 15 $\mu$l of water. A positive result indicates that the reaction was satisfactory. The cycle of thermocycling would have to be checked if the result is negative.
Matrix inhibition control (M-Inh): It is recommended to add a test in parallel to the sample to observe the possible effect of sample matrix inhibition. See protocol:

| Reaction tube | Volume ($\mu$L) |
|---|---|
| Calibrator (no. 4) | 5 |
| Sample | 5 |
| DNase-/RNase-free water | 10 |
| Final reaction volume | 20 |

An optimal result would be equal to or greater than that found in the positive control (calibrator no. 4). If total or partial inhibition is observed, the sample would have to be diluted with DNase-/RNase-free water again to prevent the effect of sample matrix inhibition.

**Interpretation of the results**

A logarithmic regression line is prepared with the calibration curve:

$$Ct = Y\ inter + slope\ x\ \log\ (number\ of\ copies)$$

Number of copies= 10 (Ct-Y inter) x slope
Finally, the following tables depict all the analyzed microorganisms, with those showing differences between cases and controls or differences between cases with a worse or better progression of the disease being indicated in bold.

Frequency ratio (cases/controls) in each GENUS

| Entity | Multiplier (95% CI) | P-value | AUC (95% CI) |
|---|---|---|---|
| **g_Lachnospirae** | **0.06 (0.01 to 0.35)** | **0.00201** | **67.1 (46.7 to 87.6)** |
| **g_Ezakiella** | **13.08 (2.16 to 79.24)** | **0.00516** | **75.0 (60.6 to 89.4)** |
| **g_Ruminococcus UCG-014** | **12.65 (2.09 to 76.62)** | **0.00578** | **68.1 (47.9 to 88.3)** |
| **g_Hungatella** | **8.57 (1.41 to 51.89)** | **0.01946** | **68.3 (51.3 to 85.4)** |
| **g_Roseburia** | **0.12 (0.02 to 0.73)** | **0.02149** | **78.1 (60.2 to 96.0)** |
| **g_[Clostridium] innocuum group** | **7.26 (1.20 to 43.99)** | **0.03100** | **67.4 (47.5 to 87.2)** |
| **g_Shuttleworthia** | **6.32 (1.04 to 38.29)** | **0.04484** | **62.4 (42.5 to 82.3)** |
| **g_Ruminococcus UCG-009** | **6.23 (1.03 to 37.71)** | **0.04662** | **62.4 (42.1 to 82.7)** |
| g_Porphyromonas | 6.00 (0.99 to 36.33) | 0.05129 | 64.3 (46.0 to 82.5) |
| g_Intestinimonas | 5.97 (0.99 to 36.16) | 0.05191 | 67.1 (48.0 to 86.3) |
| g_Bilophila | 5.93 (0.98 to 35.91) | 0.05282 | 70.2 (50.1 to 90.4) |
| g_UC5-1-2E3 | 5.85 (0.97 to 35.41) | 0.05469 | 63.1 (43.0 to 83.2) |
| g_Prevotella | 5.83 (0.96 to 35.29) | 0.05518 | 64.8 (49.0 to 80.5) |
| g_Ruminococcus UCG-007 | 5.81 (0.96 to 35.19) | 0.05557 | 62.4 (43.6 to 81.1) |
| g_Eggerthella | 5.72 (0.94 to 34.62) | 0.05786 | 63.3 (51.8 to 74.9) |
| g_Lachnospirae FCS020 group | 0.18 (0.03 to 1.10) | 0.06300 | 71.7 (52.7 to 90.7) |
| g_Ruminococcus UCG-010 | 5.39 (0.89 to 32.65) | 0.06683 | 58.6 (37.4 to 79.7) |
| g_[Ruminococcus] gnavus group | 5.25 (0.87 to 31.82) | 0.07108 | 58.8 (39.2 to 78.4) |
| g_Anaerotruncus | 5.16 (0.85 to 31.23) | 0.07433 | 71.2 (51.5 to 90.9) |
| g_Ruminiclostridium | 5.07 (0.84 to 30.67) | 0.07755 | 69.5 (50.7 to 88.4) |
| g_Turicibacter | 5.04 (0.83 to 30.51) | 0.07854 | 65.0 (47.1 to 82.9) |
| g_Ruminococcus UCG-004 | 5.00 (0.83 to 30.27) | 0.08002 | 54.5 (32.2 to 76.9) |
| g_Dielma | 4.88 (0.81 to 29.55) | 0.08461 | 69.0 (50.6 to 87.5) |
| g_Oscillibacter | 4.86 (0.80 to 29.46) | 0.08522 | 72.4 (53.1 to 91.7) |
| g_Coprococcus 3 | 0.21 (0.04 to 1.29) | 0.09327 | 66.2 (45.1 to 87.2) |
| g_Tyzzerella 3 | 0.22 (0.04 to 1.31) | 0.09549 | 59.5 (42.5 to 76.5) |
| g_Howardella | 0.22 (0.04 to 1.31) | 0.09611 | 58.1 (41.0 to 75.2) |
| g_[Eubacterium] nodatum group | 4.44 (0.73 to 26.92) | 0.10458 | 66.2 (45.6 to 86.8) |
| g_Atopobium | 0.23 (0.04 to 1.38) | 0.10700 | 62.9 (41.2 to 84.6) |
| g_Agathobacter | 0.23 (0.04 to 1.40) | 0.11035 | 61.0 (39.4 to 82.5) |
| g_Holdemanella | 4.30 (0.71 to 26.02) | 0.11273 | 56.7 (44.1 to 69.3) |
| g_CAG-352 | 0.23 (0.04 to 1.42) | 0.11406 | 58.1 (45.1 to 71.1) |
| g_Coprococcus 2 | 0.24 (0.04 to 1.46) | 0.12081 | 56.0 (38.1 to 73.8) |
| g_uncultured Thermoanaerobacterales bacterium | 4.13 (0.68 to 25.04) | 0.12250 | 63.8 (49.7 to 77.9) |
| g_Ruminococcus UCG-013 | 0.25 (0.04 to 1.48) | 0.12607 | 74.3 (55.3 to 93.3) |
| g_Angelakisella | 3.89 (0.64 to 23.54) | 0.13968 | 61.9 (45.5 to 78.4) |
| g_Veillonella | 0.26 (0.04 to 1.57) | 0.14203 | 64.8 (43.5 to 86.0) |
| g_CHKCI002 | 3.79 (0.63 to 22.97) | 0.14694 | 63.3 (51.8 to 74.9) |
| g_Papillibacter | 3.78 (0.62 to 22.88) | 0.14813 | 67.1 (47.1 to 87.2) |
| g_Propionibacterium | 3.78 (0.62 to 22.87) | 0.14826 | 63.3 (51.8 to 74.9) |
| g_Dialister | 3.73 (0.62 to 22.57) | 0.15228 | 56.7 (34.5 to 78.8) |
| g_[Ruminococcus] torques group | 3.70 (0.61 to 22.39) | 0.15485 | 69.5 (49.7 to 89.4) |
| g_Haemophilus | 0.28 (0.05 to 1.68) | 0.16306 | 60.0 (43.1 to 76.9) |
| g_Ruminococcus UCG-002 | 0.28 (0.05 to 1.69) | 0.16552 | 50.5 (28.0 to 73.0) |
| g_DTU089 | 3.52 (0.58 to 21.30) | 0.17121 | 72.4 (53.3 to 91.5) |
| g_Escherichia-Shigella | 0.30 (0.05 to 1.79) | 0.18456 | 59.8 (38.1 to 81.4) |
| g_Methanobrevibacter | 3.37 (0.56 to 20.40) | 0.18632 | 60.5 (39.6 to 81.4) |
| g_Citrobacter | 3.34 (0.55 to 20.23) | 0.18945 | 56.7 (47.8 to 65.6) |
| g_Parasutterella | 3.31 (0.55 to 20.05) | 0.19265 | 53.1 (30.5 to 75.7) |

(continued)

| Entity | Multiplier (95% CI) | P-value | AUC (95% CI) |
|---|---|---|---|
| g_Oscillospira | 3.29 (0.54 to 19.92) | 0.19516 | 59.5 (41.0 to 78.0) |
| g_Campylobacter | 3.29 (0.54 to 19.91) | 0.19540 | 63.3 (51.8 to 74.9) |
| g_Coriobacteriaceae UCG-002 | 3.23 (0.53 to 19.56) | 0.20199 | 60.2 (46.8 to 73.7) |
| g_Defluviitaleaceae UCG-011 | 3.22 (0.53 to 19.49) | 0.20335 | 63.8 (43.5 to 84.1) |
| g_Klebsiella | 0.31 (0.05 to 1.88) | 0.20382 | 57.9 (39.9 to 75.8) |
| g_metagenome | 0.31 (0.05 to 1.89) | 0.20560 | 54.8 (38.4 to 71.1) |
| g_Ruminococcus 2 | 3.18 (0.52 to 19.24) | 0.20841 | 57.4 (35.5 to 79.2) |
| g_Megasphaera | 0.32 (0.05 to 1.93) | 0.21425 | 53.8 (36.4 to 71.2) |
| g_Ruminococcus 1 | 3.10 (0.51 to 18.76) | 0.21859 | 63.1 (42.0 to 84.2) |
| g_Family XIII UCG-001 | 0.34 (0.06 to 2.05) | 0.23904 | 69.0 (48.6 to 89.5) |
| g_Solobacterium | 2.93 (0.48 to 17.75) | 0.24207 | 64.3 (43.5 to 85.1) |
| g_Synergistes | 2.91 (0.48 to 17.62) | 0.24529 | 56.7 (47.8 to 65.6) |
| g_Ruminiclostridium 1 | 2.90 (0.48 to 17.56) | 0.24683 | 60.0 (41.4 to 78.6) |
| g_UBA1819 | 2.88 (0.48 to 17.44) | 0.24988 | 66.7 (46.3 to 87.0) |
| g_Paraprevotella | 0.35 (0.06 to 2.11) | 0.25230 | 59.0 (39.1 to 79.0) |
| g_Coprococcus 1 | 2.84 (0.47 to 17.21) | 0.25570 | 62.1 (41.8 to 82.5) |
| g_Prevotella 6 | 2.84 (0.47 to 17.21) | 0.25579 | 60.0 (49.5 to 70.5) |
| g_Peptoniphilus | 2.82 (0.47 to 17.10) | 0.25859 | 60.7 (47.3 to 74.1) |
| g_[Eubacterium] fissicatena group | 2.82 (0.47 to 17.06) | 0.25974 | 66.4 (46.9 to 86.0) |
| g_S5-A14a | 2.80 (0.46 to 16.98) | 0.26194 | 60.0 (49.5 to 70.5) |
| g_Prevotella 2 | 2.80 (0.46 to 16.93) | 0.26327 | 56.7 (47.8 to 65.6) |
| g_Sellimonas | 2.74 (0.45 to 16.58) | 0.27306 | 55.2 (35.8 to 74.7) |
| g_Peptococcus | 2.72 (0.45 to 16.50) | 0.27554 | 58.8 (41.3 to 76.3) |
| g_Collinsella | 0.37 (0.06 to 2.25) | 0.28110 | 56.4 (34.4 to 78.4) |
| g_Kluyvera | 0.37 (0.06 to 2.25) | 0.28189 | 57.1 (47.6 to 66.7) |
| g_[Eubacterium] eligens group | 2.68 (0.44 to 16.22) | 0.28380 | 56.2 (33.8 to 78.6) |
| g_Prevotella 9 | 0.37 (0.06 to 2.27) | 0.28516 | 51.9 (36.4 to 67.5) |
| g_Erysipelotrichaceae UCG-004 | 0.38 (0.06 to 2.30) | 0.29163 | 60.7 (49.6 to 71.9) |
| g_Holdemania | 2.63 (0.43 to 15.95) | 0.29201 | 65.0 (43.9 to 86.1) |
| g_Azospirillum sp. 47_25 | 2.62 (0.43 to 15.86) | 0.29496 | 60.0 (49.5 to 70.5) |
| g_Oxalobacter | 2.61 (0.43 to 15.82) | 0.29601 | 61.0 (41.1 to 80.8) |
| g_Subdoligranulum | 0.38 (0.06 to 2.33) | 0.29838 | 61.0 (38.8 to 83.1) |
| g_Odoribacter | 2.58 (0.43 to 15.60) | 0.30326 | 56.0 (33.5 to 78.5) |
| g_Ruminococcus UCG-003 | 0.40 (0.07 to 2.40) | 0.31295 | 52.4 (30.6 to 74.2) |
| g_Flavonifractor | 2.44 (0.40 to 14.78) | 0.33159 | 63.3 (42.4 to 84.2) |
| g_Negativicoccus | 2.39 (0.39 to 14.48) | 0.34275 | 60.0 (49.5 to 70.5) |
| g_Butyricimonas | 2.39 (0.39 to 14.47) | 0.34336 | 57.6 (36.0 to 79.2) |
| g_Romboutsia | 2.39 (0.39 to 14.45) | 0.34410 | 50.7 (28.4 to 73.0) |
| g_CandidatusSaccharimonas | 2.38 (0.39 to 14.42) | 0.34520 | 59.8 (46.2 to 73.3) |
| g_Negativibacillus | 0.42 (0.07 to 2.56) | 0.34939 | 50.5 (28.3 to 72.7) |
| g_Anaerofustis | 2.36 (0.39 to 14.27) | 0.35114 | 56.4 (36.5 to 76.4) |
| g_Slackia | 2.34 (0.39 to 14.15) | 0.35574 | 54.8 (35.0 to 74.5) |
| g_Senegalimassilia | 0.43 (0.07 to 2.63) | 0.36338 | 54.3 (37.9 to 70.7) |
| g_Sutterella | 2.29 (0.38 to 13.89) | 0.36630 | 53.8 (32.7 to 74.9) |
| g Tyzzerella | 2.25 (0.37 to 13.63) | 0.37733 | 54.3 (36.5 to 72.0) |
| g_Ruminococcus UCG-005 | 0.45 (0.07 to 2.73) | 0.38516 | 51.9 (28.8 to 75.0) |
| g Alloprevotella | 0.45 (0.07 to 2.73) | 0.38556 | 57.1 (47.6 to 66.7) |
| g_Desulfovibrio | 2.22 (0.37 to 13.44) | 0.38586 | 54.8 (33.7 to 75.9) |
| g_Rikenella | 0.46 (0.08 to 2.77) | 0.39460 | 58.6 (43.3 to 73.8) |
| g_Enorma | 2.18 (0.36 to 13.23) | 0.39512 | 56.2 (43.5 to 68.9) |

(continued)

| Entity | Multiplier (95% CI) | P-value | AUC (95% CI) |
|---|---|---|---|
| g Ruminococcus UCG-008 | 0.46 (0.08 to 2.80) | 0.40058 | 57.1 (47.6 to 66.7) |
| g_Lactococcus | 2.16 (0.36 to 13.08) | 0.40187 | 56.4 (36.4 to 76.4) |
| g_Pseudoflavonifractor | 2.16 (0.36 to 13.08) | 0.40192 | 57.1 (44.6 to 69.7) |
| g_CAG-873 | 2.14 (0.35 to 12.98) | 0.40660 | 53.3 (46.8 to 59.9) |
| g_GCA-900066755 | 2.13 (0.35 to 12.88) | 0.41133 | 57.6 (39.5 to 75.7) |
| g_Parabacteroides | 2.12 (0.35 to 12.85) | 0.41276 | 75.7 (56.5 to 94.9) |
| g_Lachnoclostridium | 2.11 (0.35 to 12.79) | 0.41592 | 63.8 (42.6 to 85.0) |
| g_Acetanaerobacterium | 2.11 (0.35 to 12.77) | 0.41678 | 57.6 (39.3 to 75.9) |
| g_GCA-900066225 | 2.10 (0.35 to 12.74) | 0.41852 | 57.6 (36.0 to 79.2) |
| g_Anaerofilum | 2.10 (0.35 to 12.69) | 0.42072 | 54.8 (34.0 to 75.6) |
| g_Ruminiclostridium 5 | 2.03 (0.34 to 12.32) | 0.43976 | 68.6 (48.2 to 89.0) |
| g_Fusobacterium | 2.03 (0.34 to 12.30) | 0.44059 | 55.0 (38.8 to 71.2) |
| g_Anaerococcus | 2.00 (0.33 to 12.12) | 0.45013 | 57.1 (44.6 to 69.7) |
| g_Blautia | 1.99 (0.33 to 12.05) | 0.45410 | 64.3 (42.9 to 85.7) |
| g Prevotella 7 | 1.93 (0.32 to 11.70) | 0.47371 | 53.3 (46.8 to 59.9) |
| g_Lachnospirae ND3007 group | 0.52 (0.09 to 3.15) | 0.47711 | 53.8 (36.4 to 71.2) |
| g Christensenellaceae R-7 group | 1.90 (0.31 to 11.53) | 0.48327 | 56.7 (34.6 to 78.8) |
| g_[Eubacterium] brachy group | 0.53 (0.09 to 3.19) | 0.48492 | 59.0 (37.8 to 80.3) |
| g_Granulicatella | 1.89 (0.31 to 11.43) | 0.48929 | 61.0 (40.0 to 81.9) |
| g_Murdochiella | 1.89 (0.31 to 11.42) | 0.49017 | 56.7 (47.8 to 65.6) |
| g_[Eubacterium] coprostanoligenes group | 0.53 (0.09 to 3.22) | 0.49191 | 51.9 (29.1 to 74.7) |
| g_Lachnospirae UCG-010 | 0.53 (0.09 to 3.23) | 0.49330 | 51.0 (29.0 to 72.9) |
| g_Lachnospirae UCG-008 | 1.87 (0.31 to 11.30) | 0.49756 | 60.0 (44.4 to 75.6) |
| g_[Eubacterium] ventriosum group | 1.86 (0.31 to 11.26) | 0.49948 | 50.2 (26.7 to 73.7) |
| g_Lachnospira | 1.86 (0.31 to 11.24) | 0.50086 | 54.5 (33.2 to 75.9) |
| g_Prevotellaceae UCG-001 | 0.54 (0.09 to 3.27) | 0.50303 | 54.0 (39.7 to 68.4) |
| g_Pseudomonas | 1.85 (0.30 to 11.17) | 0.50504 | 53.3 (46.8 to 59.9) |
| g_Arcanobacterium | 1.85 (0.30 to 11.17) | 0.50505 | 56.7 (47.8 to 65.6) |
| g_Erysipelatoclostridium | 1.84 (0.30 to 11.15) | 0.50638 | 57.9 (35.9 to 79.9) |
| g_Erysipelotrichaceae UCG-003 | 0.55 (0.09 to 3.31) | 0.51030 | 58.6 (37.1 to 80.0) |
| g_Lachnospirae UCG-001 | 0.55 (0.09 to 3.31) | 0.51177 | 55.0 (37.0 to 73.0) |
| g_Rikenellaceae RC9 gut group | 1.83 (0.30 to 11.07) | 0.51178 | 53.1 (41.7 to 64.4) |
| g_Gordonibacter | 1.83 (0.30 to 11.06) | 0.51201 | 56.7 (38.5 to 74.9) |
| g_Neisseria | 0.55 (0.09 to 3.32) | 0.51267 | 57.1 (47.6 to 66.7) |
| g_Sanguibacteroides | 1.75 (0.29 to 10.62) | 0.54121 | 53.3 (39.1 to 67.5) |
| g_Ruminococcus V9D2013 group | 1.74 (0.29 to 10.55) | 0.54594 | 55.2 (39.9 to 70.6) |
| g_Firmicutes bacterium CAG:822 | 0.57 (0.09 to 3.48) | 0.54605 | 53.6 (46.6 to 60.6) |
| g_Abiotrophia | 1.72 (0.28 to 10.43) | 0.55406 | 53.3 (39.1 to 67.5) |
| g_[Eubacterium] ruminantium group | 1.71 (0.28 to 10.33) | 0.56119 | 58.6 (42.6 to 74.5) |
| g_Faecalibacterium | 0.59 (0.10 to 3.56) | 0.56337 | 63.3 (42.1 to 84.5) |
| g_Mobiluncus | 1.68 (0.28 to 10.16) | 0.57372 | 56.2 (43.5 to 68.9) |
| g_Bulleidia | 1.67 (0.28 to 10.14) | 0.57489 | 56.7 (47.8 to 65.6) |
| g_Candidatus Saccharibacteria bacterium UB2523 | 1.66 (0.27 to 10.04) | 0.58252 | 54.8 (34.3 to 75.2) |
| g_Adlercreutzia | 1.65 (0.27 to 10.02) | 0.58374 | 53.8 (34.0 to 73.6) |
| g_Catenibacterium | 0.61 (0.10 to 3.70) | 0.59259 | 51.9 (34.7 to 69.1) |
| g_[Bacteroides] pectinophilus group | 1.63 (0.27 to 9.90) | 0.59272 | 53.3 (46.8 to 59.9) |
| g_Eubacterium | 1.62 (0.27 to 9.79) | 0.60090 | 53.1 (41.7 to 64.4) |
| g_Cryptobacterium | 1.62 (0.27 to 9.79) | 0.60142 | 56.7 (47.8 to 65.6) |
| g_Coprobacter | 1.61 (0.27 to 9.76) | 0.60389 | 56.4 (36.1 to 76.8) |
| g_Butyricicoccus | 0.62 (0.10 to 3.77) | 0.60515 | 58.6 (37.0 to 80.2) |

(continued)

| Entity | Multiplier (95% CI) | P-value | AUC (95% CI) |
|---|---|---|---|
| g_Ruminiclostridium 9 | 1.59 (0.26 to 9.61) | 0.61534 | 62.4 (40.4 to 84.3) |
| g_Gemella | 0.63 (0.10 to 3.82) | 0.61566 | 53.8 (31.8 to 75.8) |
| g_Parvimonas | 1.59 (0.26 to 9.60) | 0.61598 | 51.4 (35.9 to 67.0) |
| g_Phocea | 0.63 (0.10 to 3.83) | 0.61803 | 54.3 (35.6 to 73.0) |
| g_Coprobacillus | 1.58 (0.26 to 9.58) | 0.61808 | 58.1 (40.0 to 76.2) |
| g_Mailhella | 1.56 (0.26 to 9.42) | 0.63053 | 53.3 (46.8 to 59.9) |
| g_[Eubacterium] oxidoreducens group | 0.65 (0.11 to 3.92) | 0.63601 | 57.1 (47.6 to 66.7) |
| g_Catabacter | 1.54 (0.25 to 9.31) | 0.63944 | 51.7 (31.1 to 72.3) |
| g_Hydrogenoanaerobacterium | 0.65 (0.11 to 3.95) | 0.64113 | 54.0 (39.6 to 68.5) |
| g_Acetitomaculum | 0.65 (0.11 to 3.95) | 0.64129 | 53.6 (46.6 to 60.6) |
| g_unculturedErysipelotrichaceae bacterium | 0.65 (0.11 to 3.95) | 0.64270 | 53.6 (46.6 to 60.6) |
| g_Actinomyces | 1.53 (0.25 to 9.27) | 0.64293 | 62.4 (40.7 to 84.1) |
| g_Lachnospirae UCG-003 | 1.51 (0.25 to 9.15) | 0.65354 | 53.3 (46.8 to 59.9) |
| g_Sneathia | 1.50 (0.25 to 9.06) | 0.66081 | 53.6 (42.3 to 64.9) |
| g_Catenisphaera | 0.67 (0.11 to 4.08) | 0.66757 | 53.6 (46.6 to 60.6) |
| g_Johnsonella | 1.48 (0.24 to 8.94) | 0.67165 | 52.9 (38.6 to 67.1) |
| g_Phascolarctobacterium | 1.48 (0.24 to 8.93) | 0.67220 | 50.7 (28.7 to 72.8) |
| g_Raoultibacter | 1.47 (0.24 to 8.92) | 0.67306 | 53.1 (41.7 to 64.4) |
| g_Peptostreptococcus | 1.47 (0.24 to 8.92) | 0.67332 | 53.6 (42.3 to 64.9) |
| g_Lachnospirae NK4A136 group | 0.68 (0.11 to 4.12) | 0.67521 | 52.4 (30.0 to 74.8) |
| g_Candidatus Soleaferrea | 1.46 (0.24 to 8.84) | 0.68066 | 50.2 (27.7 to 72.8) |
| g_Bifidobacterium | 1.45 (0.24 to 8.81) | 0.68324 | 53.3 (31.2 to 75.5) |
| g_Merdibacter | 0.69 (0.11 to 4.16) | 0.68340 | 55.2 (34.7 to 75.8) |
| g_Pygmaiobacter | 1.45 (0.24 to 8.79) | 0.68489 | 52.6 (36.3 to 69.0) |
| g_Scardovia | 1.44 (0.24 to 8.74) | 0.68990 | 53.1 (41.7 to 64.4) |
| g_Faecalicoccus | 0.70 (0.12 to 4.24) | 0.69801 | 53.3 (41.7 to 64.9) |
| g_Acidaminococcus | 1.42 (0.24 to 8.63) | 0.70003 | 54.3 (35.8 to 72.8) |
| g_Fusicatenibacter | 1.41 (0.23 to 8.56) | 0.70641 | 56.7 (34.2 to 79.1) |
| g_Roseburia | 0.71 (0.12 to 4.33) | 0.71496 | 52.9 (30.2 to 75.5) |
| g_Marvinbryantia | 1.39 (0.23 to 8.40) | 0.72166 | 51.9 (31.6 to 72.2) |
| g Enterobacter | 1.39 (0.23 to 8.40) | 0.72213 | 53.1 (41.7 to 64.4) |
| g_GCA-900066575 | 1.38 (0.23 to 8.38) | 0.72401 | 52.4 (30.4 to 74.4) |
| g_Cardiobacterium | 0.73 (0.12 to 4.42) | 0.73267 | 53.6 (46.6 to 60.6) |
| g_Staphylococcus | 0.73 (0.12 to 4.42) | 0.73267 | 53.6 (46.6 to 60.6) |
| g_uncultured Erysipelotrichia bacterium | 1.36 (0.23 to 8.27) | 0.73497 | 53.3 (46.8 to 59.9) |
| g_Fournierella | 1.36 (0.22 to 8.24) | 0.73739 | 51.0 (29.2 to 72.7) |
| g_TM7 bacterium human oral taxon HOT-869 | 1.36 (0.22 to 8.23) | 0.73843 | 53.3 (46.8 to 59.9) |
| g_Gardnerella | 0.74 (0.12 to 4.50) | 0.74729 | 53.3 (41.7 to 64.9) |
| g_Camobacterium | 1.34 (0.22 to 8.13) | 0.74862 | 53.3 (46.8 to 59.9) |
| g_Intestinibacter | 1.34 (0.22 to 8.12) | 0.74925 | 53.3 (46.8 to 59.9) |
| g_Corynebacterium 1 | 0.75 (0.12 to 4.51) | 0.74933 | 53.6 (46.6 to 60.6) |
| g_[Eubacterium] xylanophilum group | 1.34 (0.22 to 8.09) | 0.75213 | 52.9 (30.9 to 74.8) |
| g_Streptococcus | 1.33 (0.22 to 8.04) | 0.75772 | 54.3 (31.8 to 76.8) |
| g_Bacteroides | 1.33 (0.22 to 8.02) | 0.75942 | 61.0 (39.7 to 82.2) |
| g_Anaerostipes | 1.32 (0.22 to 8.02) | 0.75952 | 56.2 (34.5 to 77.9) |
| g_Tyzzerella 4 | 1.32 (0.22 to 8.00) | 0.76150 | 50.0 (36.9 to 63.1) |
| g_TM7 phylum sp. oral clone FR058 | 1.31 (0.22 to 7.96) | 0.76612 | 51.0 (33.7 to 68.2) |
| g_uncultured Candidatus Saccharibacteria bacterium | 1.31 (0.22 to 7.95) | 0.76719 | 50.5 (31.9 to 69.0) |
| g_Lactobacillus | 0.76 (0.13 to 4.62) | 0.76758 | 53.8 (34.2 to 73.4) |
| g_Clostridioides | 1.30 (0.22 to 7.90) | 0.77251 | 53.3 (46.8 to 59.9) |

(continued)

| Entity | Multiplier (95% CI) | P-value | AUC (95% CI) |
|---|---|---|---|
| g_Clostridium sp. CAG:306 | 1.30 (0.22 to 7.89) | 0.77313 | 53.1 (41.7 to 64.4) |
| g_Rothia | 1.30(0.21 to 7.86) | 0.77682 | 56.2 (41.0 to 71.4) |
| g_Oribacterium | 1.29 (0.21 to 7.83) | 0.78017 | 55.7 (40.5 to 71.0) |
| g_XXX | 1.29 (0.21 to 7.81) | 0.78172 | 60.5 (39.0 to 82.0) |
| g_Succiniclasticum | 1.29 (0.21 to 7.80) | 0.78282 | 53.3 (46.8 to 59.9) |
| g_Erysipelotrichaceae UCG-006 | 0.78 (0.13 to 4.71) | 0.78476 | 50.5 (40.9 to 60.1) |
| g_Ruminiclostridium 6 | 0.78 (0.13 to 4.72) | 0.78690 | 54.3 (32.2 to 76.4) |
| g_Weissella | 0.78 (0.13 to 4.74) | 0.79003 | 51.4 (38.2 to 64.6) |
| g_Mesorhizobium | 1.27 (0.21 to 7.72) | 0.79183 | 53.3 (46.8 to 59.9) |
| g_Tropheryma | 1.27 (0.21 to 7.72) | 0.79183 | 53.3 (46.8 to 59.9) |
| g_Allisonella | 1.26 (0.21 to 7.66) | 0.79844 | 52.6 (41.2 to 64.1) |
| g_Acinetobacter | 1.25 (0.21 to 7.59) | 0.80600 | 53.3 (46.8 to 59.9) |
| g_Selenomonas 4 | 1.25 (0.21 to 7.59) | 0.80600 | 53.3 (46.8 to 59.9) |
| g_TM7 phylum sp. canine oral taxon 237 | 1.25 (0.21 to 7.59) | 0.80600 | 53.3 (46.8 to 59.9) |
| g_Prevotellaceae NK3B31 group | 1.25 (0.21 to 7.58) | 0.80700 | 51.0 (32.9 to 69.0) |
| g_Olsenella | 1.25 (0.21 to 7.56) | 0.80873 | 50.5 (31.9 to 69.0) |
| g_Lachnospirae NC2004 group | 0.80 (0.13 to 4.87) | 0.81293 | 53.6 (46.6 to 60.6) |
| g_gut metagenome | 0.80 (0.13 to 4.87) | 0.81326 | 53.8 (34.2 to 73.5) |
| g_Cetobacterium | 1.24 (0.20 to 7.48) | 0.81809 | 53.3 (46.8 to 59.9) |
| g_Flexilinea | 1.23 (0.20 to 7.44) | 0.82277 | 53.3 (46.8 to 59.9) |
| g_Protothecazopfii | 1.23 (0.20 to 7.44) | 0.82295 | 53.3 (46.8 to 59.9) |
| g_[Eubacterium] hallii group | 0.82 (0.13 to 4.95) | 0.82630 | 53.3 (35.9 to 70.7) |
| g_uncultured | 1.22 (0.20 to 7.41) | 0.82662 | 67.6 (47.2 to 88.1) |
| g_Butyrivibrio | 0.82 (0.14 to 4.95) | 0.82673 | 53.3 (41.7 to 64.9) |
| g_Dorea | 0.82 (0.14 to 4.95) | 0.82686 | 51.9 (29.8 to 74.0) |
| g_Alloscardovia | 0.82 (0.14 to 4.97) | 0.82928 | 50.5 (37.4 to 63.6) |
| g_Alistipes | 1.20 (0.20 to 7.27) | 0.84241 | 53.3 (31.3 to 75.3) |
| g_Caproiciproducens | 0.85 (0.14 to 5.12) | 0.85490 | 50.0 (30.4 to 69.6) |
| g_Enterorhabdus | 0.85 (0.14 to 5.13) | 0.85670 | 51.2 (31.1 to 71.3) |
| g_uncultured bacterium | 1.18 (0.19 to 7.12) | 0.85988 | 55.7 (33.8 to 77.6) |
| g_Megamonas | 0.86 (0.14 to 5.23) | 0.87279 | 51.0 (37.8 to 64.1) |
| g_Harryflintia | 0.87 (0.14 to 5.28) | 0.88144 | 52.9 (35.4 to 70.3) |
| g_[Ruminococcus] gauvreauii group | 0.87 (0.14 to 5.29) | 0.88237 | 51.9 (31.1 to 72.8) |
| g_Akkermansia | 1.14 (0.19 to 6.93) | 0.88309 | 55.5 (33.3 to 77.6) |
| g_Eisenbergiella | 1.14 (0.19 to 6.93) | 0.88389 | 51.2 (31.0 to 71.4) |
| g_Clostridiumsensustricto 1 | 0.88 (0.15 to 5.33) | 0.88925 | 51.9 (31.2 to 72.6) |
| g_Lachnospirae NK4B4 group | 0.89 (0.15 to 5.38) | 0.89726 | 50.5 (40.9 to 60.1) |
| g_Ruminococcus NK4A214 group | 0.90 (0.15 to 5.42) | 0.90429 | 51.4 (29.1 to 73.8) |
| g_uncultured organism | 0.90 (0.15 to 5.43) | 0.90490 | 51.2 (32.1 to 70.3) |
| g_Leuconostoc | 1.08 (0.18 to 6.56) | 0.93015 | 51.2 (32.1 to 70.3) |
| g_Cloacibacillus | 0.92 (0.15 to 5.59) | 0.93049 | 51.7 (33.6 to 69.7) |
| g_Mycoplasma | 1.08 (0.18 to 6.55) | 0.93214 | 50.0 (36.9 to 63.1) |
| g_Lachnospirae UCG-004 | 0.93 (0.15 to 5.62) | 0.93501 | 53.3 (35.9 to 70.8) |
| g_Lactonifactor | 1.07 (0.18 to 6.48) | 0.94097 | 50.0 (36.9 to 63.1) |
| g_Mogibacterium | 0.94 (0.16 to 5.69) | 0.94621 | 51.4 (36.9 to 66.0) |
| g_Moryella | 0.96 (0.16 to 5.81) | 0.96331 | 50.5 (30.4 to 70.5) |
| g_Faecalitalea | 1.03 (0.17 to 6.24) | 0.97420 | 50.0 (28.1 to 71.9) |
| g_Family XIII AD3011 group | 1.03 (0.17 to 6.23) | 0.97531 | 54.3 (32.2 to 76.3) |
| g_Anaeroplasma | 1.01 (0.17 to 6.12) | 0.99055 | 50.0 (40.4 to 59.6) |
| g_Alkalibacterium | 1.00 (0.17 to 6.06) | 1.00000 | 50.0 (50.0 to 50.0) |

(continued)

| Entity | Multiplier (95% CI) | P-value | AUC (95% CI) |
|---|---|---|---|
| g_Anaerovorax | 1.00 (0.17 to 6.06) | 1.00000 | 53.3 (46.8 to 59.9) |
| g_Cutibacterium | 1.00 (0.17 to 6.06) | 1.00000 | 50.0 (50.0 to 50.0) |
| g_Delftia | 1.00 (0.17 to 6.06) | 1.00000 | 50.0 (50.0 to 50.0) |
| g_Microbacterium | 1.00 (0.17 to 6.06) | 1.00000 | 50.0 (50.0 to 50.0) |
| g_Pantoea | 1.00 (0.17 to 6.06) | 1.00000 | 50.0 (50.0 to 50.0) |
| g_Psychrobacter | 1.00 (0.17 to 6.06) | 1.00000 | 50.0 (50.0 to 50.0) |
| g_Serratia | 1.00 (0.17 to 6.06) | 1.00000 | 50.0 (50.0 to 50.0) |

Frequency ratio (cases/controls) in each FAMILY

| Entity | Multiplier (95% CI) | P-value | AUC (95% CI) |
|---|---|---|---|
| **f_Peptococcaceae** | **11.02 (2.18 to 55.68)** | **0.00376** | **67.6 (47.6 to 87.6)** |
| **f_Porphyromonadaceae** | **6.00 (1.19 to 30.30)** | **0.03037** | **64.3 (46.0 to 82.5)** |
| **f_gut metagenome** | **0.18 (0.04 to 0.90)** | **0.03710** | **64.3 (52.0 to 76.6)** |
| f_Desulfovibrionaceae | 4.97 (0.98 to 25.12) | 0.05247 | 69.0 (48.9 to 89.2) |
| f_Eubacteriaceae | 4.83 (0.96 to 24.41) | 0.05681 | 62.6 (42.5 to 82.7) |
| f_Coriobacteriales Incertae Sedis | 4.37 (0.87 to 22.09) | 0.07437 | 62.9 (42.4 to 83.3) |
| f_Propionibacteriaceae | 3.78 (0.75 to 19.07) | 0.10812 | 63.3 (51.8 to 74.9) |
| f_Pasteurellaceae | 0.28 (0.05 to 1.40) | 0.12108 | 60.0 (43.1 to 76.9) |
| f_Methanobacteriaceae | 3.37 (0.67 to 17.01) | 0.14188 | 60.5 (39.6 to 81.4) |
| f_Campylobacteraceae | 3.29 (0.65 to 16.60) | 0.15012 | 63.3 (51.8 to 74.9) |
| f_uncultured organism | 3.25 (0.64 to 16.43) | 0.15357 | 60.2 (46.8 to 73.7) |
| f_uncultured bacterium | 3.24 (0.64 to 16.35) | 0.15536 | 55.2 (33.4 to 77.1) |
| f_Defluviitaleaceae | 3.22 (0.64 to 16.26) | 0.15740 | 63.8 (43.5 to 84.1) |
| f_Coriobacteriaceae | 0.39 (0.08 to 1.97) | 0.25455 | 55.7 (33.7 to 77.7) |
| f_Marinifilaceae | 2.53 (0.50 to 12.80) | 0.26060 | 58.1 (35.9 to 80.3) |
| f_Camobacteriaceae | 2.53 (0.50 to 12.80) | 0.26066 | 62.6 (41.8 to 83.4) |
| f_metagenome | 0.40 (0.08 to 2.00) | 0.26235 | 57.1 (47.6 to 66.7) |
| f_Fusobacteriaceae | 2.51 (0.50 to 12.67) | 0.26573 | 57.6 (40.9 to 74.4) |
| f_Peptostreptococcaceae | 2.41 (0.48 to 12.16) | 0.28758 | 50.2 (28.0 to 72.5) |
| f_Enterobacteriaceae | 0.43 (0.08 to 2.17) | 0.30605 | 60.0 (38.3 to 81.7) |
| f_Tannerellaceae | 2.12 (0.42 to 10.72) | 0.36252 | 75.7 (56.5 to 94.9) |
| f_Eggerthellaceae | 2.03 (0.40 to 10.23) | 0.39295 | 62.4 (41.0 to 83.8) |
| f_Prevotellaceae | 0.49 (0.10 to 2.50) | 0.39453 | 59.5 (37.7 to 81.3) |
| f_Family XI | 2.02 (0.40 to 10.19) | 0.39549 | 54.3 (31.9 to 76.7) |
| f_Acidaminococcaceae | 1.98 (0.39 to 10.02) | 0.40748 | 50.5 (28.4 to 72.6) |
| f_Muribaculaceae | 0.51 (0.10 to 2.55) | 0.40955 | 50.5 (32.5 to 68.5) |
| f_Clostridiales vadinBB60 group | 1.95 (0.39 to 9.85) | 0.41908 | 56.2 (36.9 to 75.5) |
| f_Christensenellaceae | 1.88 (0.37 to 9.47) | 0.44685 | 56.2 (34.1 to 78.3) |
| f_Pseudomonadaceae | 1.85 (0.37 to 9.32) | 0.45863 | 53.3 (46.8 to 59.9) |
| f_Neisseriaceae | 0.55 (0.11 to 2.77) | 0.46667 | 57.1 (47.6 to 66.7) |
| f_Firmicutes bacterium CAG:822 | 0.57 (0.11 to 2.90) | 0.50209 | 53.6 (46.6 to 60.6) |
| f_Aerococcaceae | 1.72 (0.34 to 8.70) | 0.51063 | 53.3 (39.1 to 67.5) |
| f_Veillonellaceae | 0.58 (0.12 to 2.94) | 0.51366 | 59.5 (38.0 to 81.1) |
| f_Candidatus Saccharibacteria bacterium UB2523 | 1.66 (0.33 to 8.37) | 0.54107 | 54.8 (34.3 to 75.2) |
| f_Bifidobacteriaceae | 1.56 (0.31 to 7.86) | 0.59301 | 53.3 (31.2 to 75.5) |
| f_Actinomycetaceae | 1.55 (0.31 to 7.83) | 0.59572 | 61.9 (40.3 to 83.5) |
| f_uncultured Erysipelotrichaceae bacterium | 0.65 (0.13 to 3.30) | 0.60597 | 53.6 (46.6 to 60.6) |
| f_Leptotrichiaceae | 1.50 (0.30 to 7.56) | 0.62563 | 53.6 (42.3 to 64.9) |

(continued)

| Entity | Multiplier (95% CI) | P-value | AUC (95% CI) |
|---|---|---|---|
| f_Victivallaceae | 1.45 (0.29 to 7.32) | 0.65331 | 53.3 (46.8 to 59.9) |
| f_Cardiobacteriaceae | 0.73 (0.14 to 3.69) | 0.70410 | 53.6 (46.6 to 60.6) |
| f_Staphylococcaceae | 0.73 (0.14 to 3.69) | 0.70410 | 53.6 (46.6 to 60.6) |
| f_uncultured Erysipelotrichia bacterium | 1.36 (0.27 to 6.89) | 0.70661 | 53.3 (46.8 to 59.9) |
| f_Corynebacteriaceae | 0.75 (0.15 to 3.77) | 0.72237 | 53.6 (46.6 to 60.6) |
| f_Family XIII | 1.34 (0.26 to 6.75) | 0.72578 | 53.3 (31.4 to 75.2) |
| f_Bacteroidaceae | 1.33 (0.26 to 6.69) | 0.73346 | 61.0 (39.7 to 82.2) |
| f_Puniceicoccaceae | 1.32 (0.26 to 6.67) | 0.73582 | 53.3 (46.8 to 59.9) |
| f_Streptococcaceae | 1.32 (0.26 to 6.67) | 0.73688 | 54.3 (31.8 to 76.8) |
| f_TM7 phylum sp. oral clone FR058 | 1.31 (0.26 to 6.64) | 0.74084 | 51.0 (33.7 to 68.2) |
| f_Lactobacillaceae | 0.76 (0.15 to 3.85) | 0.74245 | 53.8 (34.2 to 73.4) |
| f_Clostridium sp. CAG:306 | 1.30 (0.26 to 6.58) | 0.74855 | 53.1 (41.7 to 64.4) |
| f_Micrococcaceae | 1.30 (0.26 to 6.55) | 0.75262 | 56.2 (41.0 to 71.4) |
| f_Erysipelotrichaceae | 1.28 (0.25 to 6.44) | 0.76851 | 51.4 (29.3 to 73.6) |
| f_Cellulomonadaceae | 1.27 (0.25 to 6.44) | 0.76916 | 53.3 (46.8 to 59.9) |
| f_Rhizobiaceae | 1.27 (0.25 to 6.44) | 0.76916 | 53.3 (46.8 to 59.9) |
| f_uncultured | 0.79 (0.16 to 4.00) | 0.77705 | 54.3 (33.1 to 75.5) |
| f_Moraxellaceae | 1.25 (0.25 to 6.33) | 0.78480 | 53.3 (46.8 to 59.9) |
| f_Anaerolineaceae | 1.23 (0.24 to 6.20) | 0.80332 | 53.3 (46.8 to 59.9) |
| f_Prototheca zopfii | 1.23 (0.24 to 6.20) | 0.80352 | 53.3 (46.8 to 59.9) |
| f_Flavobacteriaceae | 1.23 (0.24 to 6.19) | 0.80543 | 52.4 (32.6 to 72.1) |
| f_Rikenellaceae | 1.21 (0.24 to 6.13) | 0.81488 | 53.3 (31.4 to 75.3) |
| f_Leuconostocaceae | 0.85 (0.17 to 4.27) | 0.83997 | 51.9 (31.7 to 72.1) |
| f_Saccharimonadaceae | 1.17 (0.23 to 5.92) | 0.84772 | 55.7 (33.7 to 77.7) |
| f_Burkholderiaceae | 1.17 (0.23 to 5.89) | 0.85306 | 50.2 (28.1 to 72.4) |
| f_XXX | 0.87 (0.17 to 4.38) | 0.86258 | 50.0 (27.8 to 72.2) |
| f_Akkermansiaceae | 1.14 (0.23 to 5.78) | 0.87011 | 55.5 (33.3 to 77.6) |
| f_Lachnospirae | 0.88 (0.17 to 4.43) | 0.87317 | 59.0 (37.0 to 81.0) |
| f_Clostridiaceae 1 | 0.88 (0.17 to 4.44) | 0.87695 | 51.9 (31.2 to 72.6) |
| f_Ruminococcus | 1.12 (0.22 to 5.64) | 0.89314 | 62.4 (40.8 to 84.0) |
| f_Synergistaceae | 1.09 (0.22 to 5.52) | 0.91376 | 50.7 (32.5 to 68.9) |
| f_Mycoplasmataceae | 1.08 (0.21 to 5.46) | 0.92456 | 50.0 (36.9 to 63.1) |
| f_Roseburiaceae | 1.07 (0.21 to 5.42) | 0.93193 | 51.9 (29.1 to 74.7) |
| f_Atopobiaceae | 1.04 (0.21 to 5.24) | 0.96391 | 51.9 (30.0 to 73.8) |
| f_Anaeroplasmataceae | 1.01 (0.20 to 5.11) | 0.98949 | 50.0 (40.4 to 59.6) |
| f_Microbacteriaceae | 1.00 (0.20 to 5.05) | 1.00000 | 50.0 (50.0 to 50.0) |

| Frequency ratio (cases/controls) in each ORDER | | | |
|---|---|---|---|
| Entity | Multiplier (95% CI) | P-value | AUC (95% CI) |
| **o_Rhodospirillales** | **5.65 (1.11 to 28.89)** | **0.03794** | **61.4 (42.2 to 80.6)** |
| o_Desulfovibrionales | 4.97 (0.97 to 25.42) | 0.05451 | 69.0 (48.9 to 89.2) |
| o_DTU014 | 4.03 (0.79 to 20.60) | 0.09466 | 60.5 (42.3 to 78.6) |
| o_Propionibacteriales | 3.78 (0.74 to 19.30) | 0.11105 | 63.3 (51.8 to 74.9) |
| o_Pasteurellales | 0.28 (0.05 to 1.42) | 0.12415 | 60.0 (43.1 to 76.9) |
| o_Gastranaerophilales | 3.54 (0.69 to 18.12) | 0.12905 | 58.8 (39.1 to 78.5) |
| o_Methanobacteriales | 3.37 (0.66 to 17.22) | 0.14514 | 60.5 (39.6 to 81.4) |
| o_Campylobacterales | 3.29 (0.64 to 16.80) | 0.15345 | 63.3 (51.8 to 74.9) |
| o_Mollicutes RF39 | 0.38 (0.07 to 1.94) | 0.24514 | 58.6 (39.5 to 77.6) |

(continued)

| Entity | Multiplier (95% CI) | P-value | AUC (95% CI) |
|---|---|---|---|
| o_Fusobacteriales | 2.56 (0.50 to 13.06) | 0.26018 | 58.1 (40.1 to 76.1) |
| o_Enterobacteriales | 0.43 (0.08 to 2.19) | 0.30986 | 60.0 (38.3 to 81.7) |
| o_XXX | 0.47 (0.09 to 2.40) | 0.36375 | 59.5 (37.8 to 81.2) |
| o_Selenomonadales | 0.48 (0.09 to 2.45) | 0.37842 | 63.8 (42.7 to 84.9) |
| o_Pseudomonadales | 1.85 (0.36 to 9.43) | 0.46189 | 53.3 (46.8 to 59.9) |
| o_Izimaplasmatales | 1.82 (0.36 to 9.31) | 0.47168 | 54.5 (38.3 to 70.7) |
| o_Bacillales | 0.60 (0.12 to 3.07) | 0.54002 | 55.2 (33.3 to 77.2) |
| o_Coriobacteriales | 1.57 (0.31 to 8.03) | 0.58786 | 62.4 (40.2 to 84.5) |
| o_Bifidobacteriales | 1.56 (0.30 to 7.95) | 0.59585 | 53.3 (31.2 to 75.5) |
| o_Saccharimonadales | 1.55 (0.30 to 7.94) | 0.59736 | 55.2 (33.2 to 77.3) |
| o_Actinomycetales | 1.55 (0.30 to 7.92) | 0.59855 | 61.9 (40.3 to 83.5) |
| o_Victivallales | 1.45 (0.28 to 7.41) | 0.65579 | 53.3 (46.8 to 59.9) |
| o_Cardiobacteriales | 0.73 (0.14 to 3.73) | 0.70625 | 53.6 (46.6 to 60.6) |
| o_Corynebacteriales | 0.75 (0.15 to 3.81) | 0.72441 | 53.6 (46.6 to 60.6) |
| o_Micrococcales | 1.33 (0.26 to 6.80) | 0.73168 | 56.7 (39.7 to 73.6) |
| o_Lactobacillales | 1.33 (0.26 to 6.77) | 0.73538 | 54.8 (32.6 to 76.9) |
| o_Opitutales | 1.32 (0.26 to 6.75) | 0.73777 | 53.3 (46.8 to 59.9) |
| o_Erysipelotrichales | 1.28 (0.25 to 6.52) | 0.77023 | 51.4 (29.3 to 73.6) |
| o_Rhizobiales | 1.27 (0.25 to 6.51) | 0.77087 | 53.3 (46.8 to 59.9) |
| o_Bacteroidales | 1.26 (0.25 to 6.42) | 0.78380 | 62.4 (39.9 to 84.8) |
| o_Anaerolineales | 1.23 (0.24 to 6.28) | 0.80479 | 53.3 (46.8 to 59.9) |
| o_Flavobacteriales | 1.23 (0.24 to 6.27) | 0.80688 | 52.4 (32.6 to 72.1) |
| o_Chloroplast | 0.85 (0.17 to 4.35) | 0.84614 | 52.4 (38.1 to 66.7) |
| o_Betaproteobacteriales | 1.16 (0.23 to 5.92) | 0.85939 | 50.2 (28.1 to 72.4) |
| o_Verrucomicrobiales | 1.14 (0.22 to 5.85) | 0.87109 | 55.5 (33.3 to 77.6) |
| o_Synergistales | 1.09 (0.21 to 5.59) | 0.91441 | 50.7 (32.5 to 68.9) |
| o_Mycoplasmatales | 1.08 (0.21 to 5.53) | 0.92514 | 50.0 (36.9 to 63.1) |
| o_Clostridiales | 1.05 (0.21 to 5.37) | 0.95273 | 55.2 (33.0 to 77.5) |
| o_Anaeroplasmatales | 1.01 (0.20 to 5.17) | 0.98957 | 50.0 (40.4 to 59.6) |

Frequency ratio (cases/controls) in each CLASS

| Entity | Multiplier (95% CI) | P-value | AUC (95% CI) |
|---|---|---|---|
| **c_Alphaproteobacteria** | **5.66 (1.15 to 27.89)** | **0.03410** | **61.4 (42.2 to 80.6)** |
| **c_Deltaproteobacteria** | **4.97 (1.01 to 24.52)** | **0.04970** | **69.0 (48.9 to 89.2)** |
| c_Melainabacteria | 3.54 (0.72 to 17.48) | 0.12110 | 58.8 (39.1 to 78.5) |
| c_Methanobacteria | 3.37 (0.68 to 16.61) | 0.13674 | 60.5 (39.6 to 81.4) |
| c_Campylobacteria | 3.29 (0.67 to 16.20) | 0.14484 | 63.3 (51.8 to 74.9) |
| c_Fusobacteriia | 2.56 (0.52 to 12.60) | 0.24998 | 58.1 (40.1 to 76.1) |
| c_Negativicutes | 0.48 (0.10 to 2.37) | 0.36806 | 63.8 (42.7 to 84.9) |
| c_Coriobacteriia | 1.57 (0.32 to 7.74) | 0.57963 | 62.4 (40.2 to 84.5) |
| c_Saccharimonadia | 1.55 (0.31 to 7.65) | 0.58927 | 55.2 (33.2 to 77.3) |
| c_XXX | 0.67 (0.14 to 3.32) | 0.62863 | 58.8 (36.8 to 80.8) |
| c_Lentisphaeria | 1.45 (0.29 to 7.15) | 0.64867 | 53.3 (46.8 to 59.9) |
| c_Gammaproteobacteria | 0.74 (0.15 to 3.65) | 0.71205 | 57.6 (35.6 to 79.6) |
| c_Bacilli | 1.32 (0.27 to 6.52) | 0.73133 | 54.8 (32.6 to 76.9) |
| c_Erysipelotrichia | 1.28 (0.26 to 6.29) | 0.76527 | 51.4 (29.3 to 73.6) |
| c_Bacteroidia | 1.26 (0.26 to 6.20) | 0.77861 | 62.4 (39.9 to 84.8) |
| c_Anaerolineae | 1.23 (0.25 to 6.06) | 0.80055 | 53.3 (46.8 to 59.9) |

(continued)

| Entity | Multiplier (95% CI) | P-value | AUC (95% CI) |
|---|---|---|---|
| c_Oxyphotobacteria | 0.85 (0.17 to 4.19) | 0.84277 | 52.4 (38.1 to 66.7) |
| c_Verrucomicrobiae | 1.15 (0.23 to 5.65) | 0.86735 | 55.5 (33.3 to 77.6) |
| c_Actinobacteria | 0.88 (0.18 to 4.34) | 0.87485 | 52.9 (30.7 to 75.0) |
| c_Synergistia | 1.09 (0.22 to 5.39) | 0.91251 | 50.7 (32.5 to 68.9) |
| c_Mollicutes | 0.92 (0.19 to 4.52) | 0.91487 | 53.3 (31.5 to 75.2) |
| c_Clostridia | 1.05 (0.21 to 5.18) | 0.95158 | 55.2 (33.0 to 77.5) |

Frequency ratio (cases/controls) in each DIVISION

| Entity | Multiplier (95% CI) | P-value | AUC (95% CI) |
|---|---|---|---|
| p_Cyanobacteria | 3.40 (0.64 to 18.02) | 0.15190 | 61.9 (41.5 to 82.3) |
| p_Euryarchaeota | 3.37 (0.64 to 17.85) | 0.15501 | 60.5 (39.6 to 81.4) |
| p_Epsilonbacteraeota | 3.29 (0.62 to 17.42) | 0.16351 | 63.3 (51.8 to 74.9) |
| p_Fusobacteria | 2.56 (0.48 to 13.54) | 0.27147 | 58.1 (40.1 to 76.1) |
| p_Patescibacteria | 1.55 (0.29 to 8.23) | 0.60568 | 55.2 (33.2 to 77.3) |
| p_XXX | 0.65 (0.12 to 3.42) | 0.60707 | 60.7 (38.9 to 82.5) |
| p_Lentisphaerae | 1.45 (0.27 to 7.68) | 0.66307 | 53.3 (46.8 to 59.9) |
| p_Bacteroidetes | 1.26 (0.24 to 6.66) | 0.78806 | 62.4 (39.9 to 84.8) |
| p_Chloroflexi | 1.23 (0.23 to 6.51) | 0.80909 | 53.3 (46.8 to 59.9) |
| p_Verrucomicrobia | 1.15 (0.22 to 6.07) | 0.87310 | 55.5 (33.3 to 77.6) |
| p_Synergistetes | 1.09 (0.21 to 5.80) | 0.91632 | 50.7 (32.5 to 68.9) |
| p_Tenericutes | 0.92 (0.17 to 4.86) | 0.91857 | 53.3 (31.5 to 75.2) |
| p_Actinobacteria | 1.07 (0.20 to 5.65) | 0.93985 | 51.9 (29.2 to 74.6) |
| p_Proteobacteria | 0.94 (0.18 to 4.99) | 0.94403 | 58.1 (36.2 to 80.0) |
| p_Firmicutes | 1.01 (0.19 to 5.33) | 0.99473 | 51.9 (29.3 to 74.5) |

Frequency ratio (cases/controls) in each KINGDOM

| Entity | Multiplier (95% CI) | P-value | AUC (95% CI) |
|---|---|---|---|
| **k_Archaea** | **3.37 (0.90 to 12.66)** | **0.07801** | **60.5 (39.6 to 81.4)** |
| k_Eukaryota | 0.37 (0.10 to 1.38) | 0.14285 | 61.2 (45.0 to 77.3) |
| Unassigned | 0.40 (0.11 to 1.50) | 0.17910 | 61.9 (41.3 to 82.5) |
| k_Bacterium | 1.00 (0.27 to 3.75) | 0.99756 | 59.5 (37.6 to 81.4) |

**Claims**

**1.** An *in vitro* method for the prognosis of the progression of multiple sclerosis in a subject, comprising the following steps:

a. determining the levels or the concentration of bacteria belonging to the genus *Ezakiella* in an intestinal or stool sample isolated from said subject; and

b. comparing the levels or the concentration of said bacteria in said intestinal or stool sample with a reference value or with the levels or the concentration present in an intestinal or stool sample of a healthy subject, wherein an increase in the level or the concentration of bacteria belonging to the genus *Ezakiella* with respect to the reference value or with respect to the levels or the concentration present in an intestinal or stool sample of a healthy subject, is indicative of a poor progression of multiple sclerosis,

wherein poor progression is understood to be an increase in the number of relapses in patients; and wherein said levels or concentration of bacteria refer to the total amount of the bacteria belonging to the genus with respect to the total bacteria present in said sample.

**2.** The method according to claim 1, wherein the multiple sclerosis is relapsing-remitting multiple sclerosis (RRMS).

**3.** The method according to any of claims 1 to 2, wherein the levels or the concentration of bacteria belonging to the genus *Ezakiella* are determined by performing an amplification reaction from a nucleic acid preparation derived from said sample using at least one pair of primers capable of amplifying at least one representative region of said genus, and detecting the amplification product.

**4.** The method according to claim 3, wherein the amplification reaction is carried out by means of a real-time polymerase chain reaction.

**5.** An *in vitro* method for the prediction of the therapeutic response of a patient diagnosed with multiple sclerosis, preferably relapsing-remitting multiple sclerosis, wherein said method comprises the steps of determining and comparing the levels or the concentration of bacteria according to any of claims 1 to 4, and wherein an increase in the level or the concentration of bacteria belonging to the genus *Ezakiella* is indicative of a lack of response to treatment.

**6.** The method according to claim 5, wherein said treatments are selected from the list consisting of interferon beta, interferon beta 1a, interferon beta 1b, natalizumab, fingolimod, dimethyl fumarate, teriflunomide, and glatiramer acetate.

**7.** The method according to any of claims 1 to 6, wherein said method further comprises storing the results of the method in a data carrier, preferably wherein said data carrier is a computer readable medium.

**8.** A computer-implemented method, wherein said method comprises at least the comparative step and optionally the provision of a result as a consequence of said comparison, as these steps are defined in the method according to any of claims 1 to 6.

**9.** A method for the prognosis of the progression of multiple sclerosis in a subject according to claims 1 to 5, comprising the following steps:

a. contacting the sample to be analyzed with a reaction mixture containing specific primers capable of amplifying bacteria belonging to the genus *Ezakiella,*
b. performing amplification by means of polymerase chain reaction,
c. identifying the formation of the products of the preceding step, said formation being indicative of the levels or the concentration of bacteria belonging to *Ezakiella,* wherein an increase in the level or the concentration of bacteria belonging to the genus *Ezakiella* with respect to the reference value or with respect to the levels or the concentration present in an intestinal or stool sample of a healthy subject is indicative of a poor progression of multiple sclerosis, wherein poor progression is understood to be an increase in the number of relapses in patients.

**10.** Method for the prediction of the therapeutic response of a patient diagnosed with multiple sclerosis in a subject according to claims 5 to 6, comprising the following steps:

a. contacting the sample to be analyzed with a reaction mixture containing specific primers capable of amplifying bacteria belonging to the genus *Ezakiella,*
b. performing amplification by means of polymerase chain reaction,
c. identifying the formation of the products of the preceding step, said formation being indicative of the levels or the concentration of bacteria belonging to *Ezakiella,* wherein an increase in the level or the concentration of bacteria belonging to the genus *Ezakiella* with respect to the reference value or with respect to the levels or the concentration present in an intestinal or stool sample of a healthy subject is indicative of a lack of response to treatment.

**11.** The method according to claim 9 or 10, wherein DNA fragments included or comprised in sequences 6 to 8 are amplified.

**12.** The method according to claims 9 to 11, wherein the amplification products which allow identifying the different bacterial species and groups are detected by means of using probes, wherein these probes preferably have a length between 15 and 25 nucleotides.

**13.** A kit capable of implementing the methodology described in any of claims 9 to 12.

**FIGURE 1**

Shannon: Genus

**FIGURE 2**

A

B.

**FIGURE 3**

PCoA - Genus - TIME

**FIGURE 4.**

Significant between cases and healthy controls)

| Median for cases | Median for controls | Difference | P-value | AUC |
|---|---|---|---|---|
| 264.0000 | 1828.0000 | 1564.0000 | 0.0107 | 0.7857 |

FIGURE 4. (Cont)

**Roseburia**

| Cut-off point | Se | Sp | J |
|---|---|---|---|
| -Inf | 1,0000 | 0,0000 | 0,0000 |
| 37.5 | 0,9286 | 0,1429 | 0,0714 |
| 75.5 | 0,9286 | 0,2143 | 0,1429 |
| 102 | 0,9286 | 0,2857 | 0,2143 |
| 163 | 0,9286 | 0,3571 | 0,2857 |
| 222 | 0,9286 | 0,4286 | 0,3571 |
| 264 | 0,9286 | 0,5000 | 0,4286 |
| 332 | 0,9286 | 0,5714 | 0,5000 |
| 389.5 | 0,9286 | 0,6429 | 0,5714 |
| 531.5 | 0,8571 | 0,6429 | 0,5000 |
| 858.5 | 0,7857 | 0,6429 | 0,4286 |
| 1099.5 | 0,7857 | 0,7143 | 0,5000 |
| 1173.5 | 0,7143 | 0,7143 | 0,4286 |
| 1228.5 | 0,6429 | 0,7143 | 0,3571 |
| 1379.5 | 0,6429 | 0,7857 | 0,4286 |
| 1609.5 | 0,6429 | 0,8571 | 0,5000 |
| 1751 | 0,5714 | 0,8571 | 0,4286 |
| 1828 | 0,5000 | 0,8571 | 0,3571 |
| 2154 | 0,4286 | 0,8571 | 0,2857 |
| 2475.5 | 0,3571 | 0,8571 | 0,2143 |
| 2647.5 | 0,2857 | 0,8571 | 0,1429 |
| 3307 | 0,2857 | 0,9286 | 0,2143 |
| 3856 | 0,2143 | 0,9286 | 0,1429 |
| 3929.5 | 0,2143 | 1,0000 | 0,2143 |
| 5132.5 | 0,1429 | 1,0000 | 0,1429 |
| 14212 | 0,0714 | 1,0000 | 0,0714 |
| Inf | 0,0000 | 1,0000 | 0,0000 |

**FIGURE 4. (Cont)**

| Median for cases | Median for controls | Difference | P-value | AUC |
|---|---|---|---|---|
| 3.0000 | 0.0000 | -3.0000 | 0.0036 | 0,7704 |

**Ezakiella**

| Cut-off point | Se | Sp | J |
|---|---|---|---|
| Inf | 1,0000 | 0,0000 | 0,0000 |
| 214 | 1,0000 | 0,0714 | 0,0714 |
| 95.5 | 1,0000 | 0,1429 | 0,1429 |
| 33 | 1,0000 | 0,2143 | 0,2143 |
| 4.5 | 1,0000 | 0,3571 | 0,3571 |
| 3.5 | 1,0000 | 0,4286 | 0,4286 |
| 2.5 | 1,0000 | 0,5714 | 0,5714 |
| 1 | 0,9286 | 0,5714 | 0,5000 |
| -Inf | 0,0000 | 1,0000 | 0,0000 |

**FIGURE 4 (cont.)**

Cut-off values with sensitivity and specificity. The best cut-off point with its corresponding sensitivity and specificity is specified in the blue line.

**FIGURE 4. (Cont)**

**Genus Ruminococcus**

| Cut-off point | Se | Sp | J |
|---|---|---|---|
| Inf | 1,0000 | 0,0000 | 0,0000 |
| 293.5 | 1,0000 | 0,0714 | 0,0714 |
| 182.5 | 1,0000 | 0,1429 | 0,1429 |
| 123 | 1,0000 | 0,2143 | 0,2143 |
| 106.5 | 1,0000 | 0,2857 | 0,2857 |
| 87.5 | 1,0000 | 0,3571 | 0,3571 |
| 79 | 0,9286 | 0,3571 | 0,2857 |
| 74 | 0,8571 | 0,3571 | 0,2143 |
| 67.5 | 0,8571 | 0,4286 | 0,2857 |
| 44 | 0,8571 | 0,5000 | 0,3571 |
| 23.5 | 0,8571 | 0,5714 | 0,4286 |
| 22 | 0,8571 | 0,6429 | 0,5000 |
| 20.5 | 0,7857 | 0,7143 | 0,5000 |
| 19.5 | 0,7143 | 0,7143 | 0,4286 |
| 18.5 | 0,6429 | 0,7143 | 0,3571 |
| 16.5 | 0,5714 | 0,7143 | 0,2857 |
| 12.5 | 0,4286 | 0,7857 | 0,2143 |
| 9.5 | 0,3571 | 0,8571 | 0,2143 |
| 8 | 0,2857 | 0,9286 | 0,2143 |
| 5.5 | 0,2143 | 0,9286 | 0,1429 |
| 2 | 0,1429 | 0,9286 | 0,0714 |
| -Inf | 0,0000 | 1,0000 | 0,0000 |

| Median for cases | Median for controls | Difference | P-value | AUC |
|---|---|---|---|---|
| 44.0000 | 15.0000 | -29.0000 | 0.0241 | 0.7526 |

**FIGURE 4 (cont.)**

## Coordinates of the Curve

Test Result Variable(s): g__CAG-56

| Positive if Greater Than or Equal To* | Sensitivity | 1 - Specificity |
|---|---|---|
| -1.0000000000 | 1.000 | 1.000 |
| .0065519350 | .786 | .429 |
| .0206933320 | .786 | .357 |
| .0104652900 | .714 | .357 |
| .0350416813 | .643 | .357 |
| .0370538359 | .643 | .286 |
| .0523122270 | .643 | .214 |
| .0734805557 | .571 | .214 |
| .0824529614 | .500 | .214 |
| .0869866382 | .500 | .143 |
| .1304220813 | .429 | .143 |
| .1705664041 | .357 | .143 |
| .2004442555 | .357 | .071 |
| .2298619617 | .286 | .071 |
| .2380657741 | .214 | .071 |
| .2577047255 | .143 | .071 |
| .2799475985 | .071 | .071 |
| .3363888406 | .000 | .071 |
| 1.0258501089 | .000 | .000 |

The test result variable(s): g__CAG-56 has at least one tie between the positive actual

Lachnospira

CAG 56

ROC Curve

Sensitivity

1 - Specificity

Diagonal segments are produced by ties

P-value 0.002 : AUC 0.67

**FIGURE 4 (cont.)**

**Bilophila**

| Median for cases | Median for controls | Difference | P-value | AUC |
|---|---|---|---|---|
| 96.0000 | 24.0000 | -72.0000 | 0.0363 | 0.7347 |

**FIGURE 4. (Cont)**

| Cut-off point | Se | Sp | J |
|---|---|---|---|
| Inf | 1,0000 | 0,0000 | 0,0000 |
| 1221 | 1,0000 | 0,0714 | 0,0714 |
| 896 | 1,0000 | 0,1429 | 0,1429 |
| 538.5 | 1,0000 | 0,2143 | 0,2143 |
| 363.5 | 1,0000 | 0,2857 | 0,2857 |
| 350 | 0,9286 | 0,2857 | 0,2143 |
| 259 | 0,8571 | 0,2857 | 0,1429 |
| 172.5 | 0,7857 | 0,2857 | 0,0714 |
| 161 | 0,7857 | 0,3571 | 0,1429 |
| 137 | 0,7857 | 0,4286 | 0,2143 |
| 119.5 | 0,7143 | 0,4286 | 0,1429 |
| 96 | 0,7143 | 0,5000 | 0,2143 |
| 74 | 0,7143 | 0,5714 | 0,2857 |
| 71.5 | 0,7143 | 0,6429 | 0,3571 |
| 63.5 | 0,7143 | 0,7143 | 0,4286 |
| 56 | 0,7143 | 0,7857 | 0,5000 |
| 52.5 | 0,7143 | 0,8571 | 0,5714 |
| 42 | 0,6429 | 0,8571 | 0,5000 |
| 31.5 | 0,5714 | 0,8571 | 0,4286 |
| 24 | 0,5000 | 0,8571 | 0,3571 |
| 18 | 0,4286 | 0,8571 | 0,2857 |
| 16 | 0,3571 | 0,8571 | 0,2143 |
| 14.5 | 0,2857 | 0,9286 | 0,2143 |
| 7 | 0,2143 | 0,9286 | 0,1429 |
| -Inf | 0,0000 | 1,0000 | 0,0000 |

**FIGURE 5**

**FIGURE 6**

Europäisches
Patentamt

European
Patent Office

Office européen
des brevets

## EUROPEAN SEARCH REPORT

Application Number

EP 19 38 2093

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MIRZA ALI ET AL: "The gut microbiome and microbial translocation in multiple sclerosis", CLINICAL IMMUNOLOGY, vol. 183, 9 March 2017 (2017-03-09), pages 213-224, XP085256987, ISSN: 1521-6616, DOI: 10.1016/J.CLIM.2017.03.001 * abstract * | 1-13 | INV. C12Q1/6883 G01N33/569 |
| A | PRÖBSTEL ANNE-KATRIN ET AL: "The Role of the Gut Microbiome in Multiple Sclerosis Risk and Progression: Towards Characterization of the "MS Microbiome"", NEUROTHERAPEUTICS, ELSEVIER INC, US, vol. 15, no. 1, 16 November 2017 (2017-11-16), pages 126-134, XP036639072, ISSN: 1933-7213, DOI: 10.1007/S13311-017-0587-Y [retrieved on 2017-11-16] * abstract * | 1-13 | |
| A | TREMLETT HELEN ET AL: "The Gut Microbiota and Pediatric Multiple Sclerosis: Recent Findings", NEUROTHERAPEUTICS, ELSEVIER INC, US, vol. 15, no. 1, 22 September 2017 (2017-09-22), pages 102-108, XP036615728, ISSN: 1933-7213, DOI: 10.1007/S13311-017-0574-3 [retrieved on 2017-09-22] * abstract * | 1-13 | TECHNICAL FIELDS SEARCHED (IPC) C12Q G01N |

-/--

The present search report has been drawn up for all claims

| Place of search | Date of completion of the search | Examiner |
|---|---|---|
| Munich | 23 October 2019 | Costa Roldán, Nuria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 693 474 A1**

Europäisches
Patentamt

European
Patent Office

Office européen
des brevets

# EUROPEAN SEARCH REPORT

Application Number

EP 19 38 2093

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FREEDMAN SAMANTHA N ET AL: "The "Gut Feeling": Breaking Down the Role of Gut Microbiome in Multiple Sclerosis", NEUROTHERAPEUTICS, ELSEVIER INC, US, vol. 15, no. 1, 4 December 2017 (2017-12-04), pages 109-125, XP036414614, ISSN: 1933-7213, DOI: 10.1007/S13311-017-0588-X [retrieved on 2017-12-04] * abstract; table 1 * | 1-13 | |
| A | NAVARRO-LOPEZ V ET AL: "MICROBIOME CORE IN PATIENTS WITH RELAPSING-REMITTING MULTIPLE SCLEROSIS", ANNALS OF NUTRITION & METABOLISM, vol. 72, no. Suppl. 1, 2018, pages 56-57, XP009516765, & 9TH WORKSHOP ON PROBIOTICS AND PREBIOTICS OF THE SPANISH-SOCIETY-OF-PROBIOTICS-AND-PREBIOTICS (SEPYP); ZARAGOZA, SPAIN; FEBRUARY 15 -16, 2018 * the whole document * | 1-13 | |

TECHNICAL FIELDS SEARCHED (IPC)

The present search report has been drawn up for all claims

| Place of search | Date of completion of the search | Examiner |
|---|---|---|
| Munich | 23 October 2019 | Costa Roldán, Nuria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

42

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JANGI S ; GANDHI R ; COX LM ; LIN ; VON GLEHN F ; YAN R ; PATEL B ; MAZZOLA MA ; LIU S ; GLANZ BL et al.** Alterations of the human gut microbiome in multiple sclerosis. *Nat Commun.*, 28 June 2016, vol. 7, 12015

- **TREMLETT H ; FADROSH DW ; FARUQI AA et al.** Gut microbiota in early pediatric multiple sclerosis: a case-control study. *Eur J Neurol,* August 2016, vol. 23 (8), 1308-1321
- **J. HOORFAR et al.** Making internal amplification control mandatory for diagnostic PCR. *J. of Clinical Microbiology,* December 2003, 5835